# EUROPEAN PATENT SPECIFICATION

(11) **EP 1 682 349 B1**
(45) Date of publication and mention of the grant of the patent: **23.04.2008**
(21) Application number: 04811003.5
(22) Date of filing: 04.11.2004
(51) Int. Cl.: B32B 27/32, C08J 5/18

(54) **CLING FILM LAMINATE STRUCTURE**
FRISCHHALTEFOLIENLAMINATSTRUKTUR
STRUCTURE LAMINEE DE PELLICULE AUTOCOLLANTE

(30) Priority: 04.11.2003 US 700761; 14.06.2004 US 867438
(43) Date of publication of application: 26.07.2006
(73) Proprietor: Bostik, Inc., Wauwatosa, Wisconsin 53226-3413 (US)
(72) Inventor: CARPER, James, D., Waukesha, WI 53186 (US); ALPER, Mark, D., Mukwonago, WI 53149 (US); SAJOT, Nicolas, Edgard, Wauwatosa, WI 53226 (US)
(74) Representative: Sanderson, James L.C.
(86) International application number: PCT/US2004/038079
(87) International publication number: WO 2005/044560

(56) References cited:
- US-A- 4 518 654
- US-B1- 6 361 875

## Description

### BACKGROUND OF THE INVENTION

The present invention relates to cling films, and more particularly to a laminate composed of a cling film layer bonded to a non-stretchable base layer, a process for making the cling film laminate, and various methods of using the cling film laminate.

The "cling" property of a polymer film is generally defined as its cohesive bonding strength, i.e. its ability to bond to itself. This cling property is also sometimes referred to as a self-adhesive property, an auto-adhesive property or a cold seal property. In any event, cling is a highly desirable property which enables polymer films having the desired cling strength to be useful in various applications, especially in the packaging and fastening industries.

Cling films have been used in multi-layer laminate structures in the past, and, in fact, cling films may themselves be multi-layer constructions. Cling film is especially well suited for use in various bundling, packaging and palletizing operations and one significant commercially important application is what is commonly referred to as "stretch wrap" film. For example, the load on a pallet may be bundled for shipping by stretch-wrapping a cling film several times around the articles stacked on the pallet. The cling film may be composed of two, three or more layers with each individual layer providing a desirable property so that the film, as a whole, possesses a desirable combination of stretch, tensile, tear resistance, puncture resistance, thermal stability and slip properties. In any event, however, when used as stretch wrap, a cling film is usually stretched as it is applied to place it under considerable tension in a face to back relationship, i.e. the front surface having cling properties engages the back surface having non-cling properties. Examples of multi-layer cling film constructions intended to be used as stretch wrap can be found in U.S. Patents 5,049,423,5,085,927 and 5,093,188.

Bullard et al U.S. Patent 5,902,684 and Eichbauer U.S. Patent 5,814,399 both disclose a multi-layered stretch wrap film intended to be stretched at least 400% from it original unstretched state. The stretch wrap film has at least four layers and is comprised of a pair of opposite outer cling layers comprising the front as well as the back surfaces of the stretch wrap film, at least one inner polymeric layer and at least one core layer. The cling layers are polyethylene copolymers comprised of ethylene copolymerized with 1 to 20 weight percent of an alpha-olefin monomer containing 3 to 12 carbon atoms These polyethylene copolymers have a relatively high density ranging from 0.88 g/cm³ to 0.94 g/cm³, and are not metallocene catalyzed. Although the inner polymeric layer is comprised of metallocene catalyzed polyethylene copolymers, this layer is disposed between the two outer cling layers and thus does not have an exposed outer surface which can provide cling properties or function as a cling layer, but instead is intended as a puncture resistant layer for the stretch wrap film.

Another application of cling films in the packaging industry is demonstrated in Walor U.S. Patent 4,905,298 and Branson U.S. Patent 4,758,099 which both describe the use of cling film as a resealable closure for sealing a flexible container such as a plastic bag. Walor utilizes a modified polyvinyl chloride film to provide the cling properties while Branson utilizes a low density polyethylene treated with an additive such as ethylene vinyl acetate to provide the cling characteristic. Once again, neither Walor nor Branson utilize metallocene catalyzed copolymers.

It is also common in the packaging industry to utilize compositions referred to as "cold seals" or "cohesives" as a bonding or fastening system. A cold seal or cohesive is generally a water-based latex adhesive which is nontacky to the touch, yet adheres to itself with pressure. Cold seals are employed for a variety of uses, particularly to bond or fasten various packaging applications such as wrappers for snack foods like candy, granola bars and potato chips, sterilizable medical packaging, self-seal and tamper-evident envelopes, banding for paper money, napkins and articles of clothing, as well as for protective packaging such as fold over "blister" packages for hardware and other small items. Additionally, cold seals have been proposed for use as release-paper-free tapes such as fastening tapes for disposable diapers.

Baetzold et al U.S. Patent 6,221,448 discloses a cold seal or cohesive adhesive composition which is formulated as a hot melt adhesive. The cold seal described in Baetzold et al utilizes one or more metallocene-catalyzed copolymers of ethylene and an alpha-olefin either alone as the cold seal adhesive itself or the copolymers may be formulated together with conventional waxes and tackifiers into a hot melt adhesive. The copolymers have a melt index ranging from 200g/10 min. to 2000g/10 min. which is appropriate for formulating a hot melt adhesive, or for applying the copolymers neat as a hot melt, but not for making a film such as a cling film, which instead should preferably be in the range of 50g/10 min. or less and more preferably 5g/10 min. or less.

As noted previously, cling films have also been used in the fastening industry. Kobe et al U.S. Patent 5,908,695 discloses a fastener system comprised of a laminate having a contact responsive fastening layer which has a surface that possesses essentially no surface tack. The fastening layer includes a polymeric material that permits multiple fastening and releasing of the fastening layer with a target surface. The target surface may comprise either another essentially tack free surface of a like laminate, or it may simply be a non-tacky smooth surface such as glass or paper. The polyethylene copolymers described therein, however, contain only relatively low amounts of comonomer (up to 15% by weight) and are not metallocene catalyzed.

The prior art is also replete with numerous examples of constructions employed as a fastening system for disposable soft goods. The phrase "disposable soft goods" refers to articles such as disposable diapers, sanitary napkins, surgical drapes, hospital gowns, hospital pads and many other utilitarian objects wherein one or more layer is composed of a nonwoven material. Examples of fastening systems for disposable soft goods can be found in numerous U.S. patents such as U.S. Patents 4,973,326; 4,894,060; 4,726,971; 4,585,450; 4,540,414; 4,296,750; and 4,210,144.

Disposable diapers of the type widely used today have included pressure sensitive adhesive tapes as fasteners for securing the diaper about the waist of an infant. Various other fastening systems have also been employed, such as combining the adhesive coated tape with a landing zone on the front panel of the diaper, as well as the use of hook and loop fasteners.

An inherent problem with the forgoing diaper fastening systems using pressure sensitive adhesive tabs is that of contamination of the tacky surfaces. Thus, talcum powder, baby oil or other foreign matter that finds its way onto either the pressure sensitive adhesive of the tab or onto the landing zone to which the pressure sensitive adhesive is adhered can reduce the reliability of the fastener, can limit the bonding strength of the adhesive, and/or can prevent fastening altogether. Although the use of hook and loop fasteners substantially overcomes the problem of reduced fastener reliability due to contaminants on pressure sensitive adhesive, hook and loop fastener systems are relatively expensive and may not be economical for use on inexpensive disposable diapers.

Disposable diapers utilizing tab fasteners which are coated with autoadhesives are also known in the art, as disclosed in Mann et al U.S. Patent 5,085,655. The tab fastener disclosed in U.S. Patent 5,085,655 is in the form of a laminate having an autoadhesive layer formed by an elastomer and a base carrier layer formed by a layer of thermoplastic material. The term "auto adhesive" is defined in the 655 patent as the self-adhesive or cohesive property of a polymer which enables the polymer to adhere to itself by application of pressure, but is substantially non-adhesive with respect to many other materials. The autoadhesive surface is formed of an elastomer comprising a block copolymer having rubbery segments and non-rubbery segments. The thermoplastic carrier layer is preferably a polyolefin such as polyethylene or polypropylene, or a polyester. Tab fasteners of the construction disclosed in the '655 patent, however, suffer from the disadvantage of having relatively high peel strength. The examples given in the '655 patent show peel strength of 53.7-80.4 kg/m (1364-2043 g/in). This relatively high peel strength makes the block copolymer based laminate of the'655 patent difficult to function as a workable refastenable tape tab. For example, when one attempts to remove fastening tabs of this type from a diaper after they have been secured in place, the autoadhesive layer will stretch or deform due to this relatively high peel strength resulting in the possibility of permanent deformation of the tab and the inability to refasten the tab if desired. This, for example, prevents a person from checking the diaper after it has been on an infant for a period of time, and then refastening the diaper if it has not been soiled. Thus, it would be desirable to provide a laminate structure which eliminates, or at least severely limits stretching and/or deformation of an autoadhesive layer. US 6,361,875 discloses a multilayer stretch cling film that may stretch 552% in the MD direction and 871% in the TD direction.

### SUMMARY OF THE INVENTION

The present invention is directed toward a unique cling film fastener structure, a process for making the cling film fastener, and use of the cling film fastener in various applications. According to the present invention there is provided cling film fastener system having a fastening component comprising a cling layer that does not stretch more than 50% having an inner surface and an outer cling surface, said cling layer comprising a polyolefin copolymer, said polyolefin copolymer selected from the group consisting of a metallocene or single site catalyzed ethylene-based copolymer of ethylene and a C₃ to C₁₈ alpha-olefin comonomer having at least 20% by weight of said comonomer, a metallocene or single site catalyzed propylene-based copolymer of propylene and a C₂ to C₁₈ alpha-olefin comonomer having at least 5% by weight of said comonomer, and a blend of the ethylene-based copolymers, the propylene-based copolymers, or one or more of said ethylene-based copolymers with one or more of the propylene-based copolymers. The cling film fastener utilizes one or more polyolefin copolymers that provide the highly desirable cling properties.

The cling film fastener has as one of its primary components a cling layer containing one or more polyolefin copolymers that provides autoadhesive or cling surface properties. In one embodiment, the cling layer may be a self-supporting sheet in the form of a monolayer film of the one or more polyolefin copolymers. In use, the cling film monolayer is bonded directly to a target surface so that its cling surface is exposed.

In another embodiment, the cling film fastener comprises a multi-layered laminate structure. In this embodiment, the cling layer has one or more polyolefin copolymers that provides autoadhesive or cling surface properties bonded to or coated on a carrier layer which provides support for the cling layer. The carrier layer itself may be a single layer or a multi-layered construction, and either, or both, of the cling layer and the carrier layer (or any or all of the individual layers of the carrier layer) may be substantially non-stretchable so as to render the entire cling film fastener itself substantially non-stretchable. The carrier layer preferably is the component of the laminate that eliminates, or substantially limits, stretching of the cling layer. The carrier layer thus preferably provides dimensional stability both in the longitudinal and/or the cross direction to prevent stretching or deformation of the cling layer. In use, the carrier layer is bonded directly to a target surface so that the cling surface of the cling layer is exposed.

The cling layer, in either its monolayer or multi-layered laminate form, will stretch no more than about 50% in either direction. More preferably, stretching should be limited to no more than about 25% from its original non-stretched configuration, and most preferably stretching in either direction should be less than 10%. Stretching and/or substantial deformation of the cling layer is undesirable as it reduces the ability of the autoadhesive surface of the polyolefin copolymers to adhere to itself. The fact that the fastener of the present invention is constructed of one or more unique polyolefin copolymers and further is substantially non-stretchable and non-deformable distinguishes the present laminate from cling films used as stretch wrap since stretch wraps typically desire at least about 200% stretchability.

The cling film fastener of the present invention is particularly useful in bonding systems and/or fastening systems for disposable soft goods, especially disposable diapers, sanitary napkins, surgical drapes, hospital gowns, hospital pads, face masks and other such objects having one or more layer composed of a nonwoven material. The fastener of the present invention is particularly useful in systems of the type including fastening tabs or tapes on the rear panel and a landing zone located on the front panel of a diaper. The fastener of the present invention may be used to provide both the fastening tabs or tapes and the reinforcing landing zone on the diapers. In such an application, the autoadhesive properties of the cling layer may help eliminate or substantially reduce the contamination problems of the prior art systems using pressure sensitive adhesives. In addition, the autoadhesive surfaces of the cling layer are substantially non-adhesive at ambient temperature with respect to the carrier layer thus enabling the fastener to be manufactured in the form of a web or roll for use in conventional diaper manufacturing systems. Also, a fastener composed of a cling layer and anonstretchable carrier layer provides the fastening system with relatively low peel, but relatively high shear strength. Preferably, when used as a disposable diaper fastening system, the peel strength is 39 kg/m (1000g/inch) or less, more preferably 24 kg/m (600g/inch) or less, and most preferably 16 kg/m (400g/inch) or less. On the other hand, the shear strength is preferably greater than 4 hours, and most preferably greater than 8 hours, as further described herein. Thus, the fastening tabs provide sufficient peel and shear forces to hold the diaper in place yet may be readily opened by a user without rupturing or significantly damaging the front panel and/or the landing zone of the disposable diaper while at the same time preventing stretching or deformation of the cling film layer itself so that the tabs may be refastened if desired.

The fastener of the present invention is also useful in various packaging applications. For example, the fastener could be used as a closure system for (1) food products, e. g. snack food wrappers, such as candy, granola bars and potato chips, (2) medical devices, e. g. sterilizable medical packages for items such as gauzes and bandages, (3) self-seal and tamper-evident envelopes, (4) banding for paper money, napkins and articles of clothing, (5) blister-type packages for various small articles, (6) closing corrugated boxes or other rigid packaging applications, and (7) closing plastic bags or other flexible packaging applications. In the above applications, the packaging material itself, i.e. the candy wrap material, the paper envelope material, etc., used to form the individual package would comprise the target surface to which the cling layer (in a monolayer form) is attached or to which the carrier layer (in a multi-layered laminate form) is attached.

As previously described, the cling layer may be a multi-layered laminate composed of a coating or layer of a polyolefin copolymer providing autoadhesive or cling surface properties bonded to a carrier layer. The carrier layer in turn may also comprise a multi-layered construction. For example, a multi-layered laminate may be constructed of a cling layer composed of one or more polyolefin copolymers, and a carrier layer comprised of a base layer (to be attached to a target surface) and a structural layer interposed between the base layer and the cling layer. In this multi-layered construction, each of the three layers are integrally bonded together, as by coextrusion, meltblown, spunbond or other adhesion techniques, to form the desired fastener of the present invention. In another form, however, the cling layer can be directly bonded onto the base layer to form the desired fastener of the present invention without the structural substrate layer. For example, in this form, the cling layer could be directly extruded onto the base layer, or the cling film layer could be applied using any known fiber forming technology such as being meltblown or spunbond directly onto the base layer. Alternately, the carrier layer could be directly formed onto the cling layer. For example, if the base layer is a nonwoven material, the nonwoven can be directly meltblown or spun bonded onto the structural substrate layer and/or the cling layer.

The material useful for forming the carrier layer is any material which is substantially non-stretchable, as defined herein, in the machine direction (longitudinally) and/or the cross machine direction (transverse). Preferably, the carrier layer is comprised of a nonwoven material, which gives the fastener a soft, cloth-like feel, or a thermoplastic film such as a polyolefin like polyethylene or polypropylene. Thus, each of the individual layers that are used to make up the carrier layer may be comprised of a nonwoven material, or a thermoplastic film such as a polyester, a polyamide, a polysulfone, an acrylic polymer, a polystyrene, a polyurethane, a polycarbonate, a halogenated polymer, a cellulosic, a polyacrylonitrile, and an ionomer based on sodium or zinc salts of ethylene/methacrylic acid, or any suitable polyolefin or combination of polyolefins such as polyethylene, polypropylene, copolymers of ethylene, copolymers of propylene, or polymers obtained from ethylene and/or propylene copolymerized with other olefins, particularly C₃ to C₁₂ olefins. Particularly preferred are nonwovens, polypropylene and linear low density polyethylene (LLDPE). Suitable LLDPEs include those having a density of between 0.90g/cm³ to 0.94g/cm³ with a melt index between 0.5g/10 min. to 30g/10 min. Suitable polypropylene is normally highly crystalline with a density range between 0.89g/cm³ to 0.91g/cm³ and a melt index between 0.1g/10 min. to 300g/10 min.

The cling layer that provides the autoadhesive or cling properties is preferably comprised of a suitable single site or metallocene catalyzed ethylene-based copolymer of ethylene and one or more C₃ to C₁₈ alpha-olefin comonomer, or a single site or metallocene catalyzed propylene-based copolymer of propylene and one or more C₂ to C₁₈ alpha-olefin comonomer, or a blend of the ethylene-based copolymers, the propylene-based copolymers, or one or more of the ethylene-based copolymers with one or more of the propylene-based copolymers. The alpha-olefin comonomer content in the ethylene-based copolymer is at least 20% by weight, and preferably from 20% to 50% by weight. The alpha-olefin comonomer content in the propylene-based copolymer is at least 5%, and preferably 5% to 30%, and most preferably 5% to 15% by weight. Suitable ethylene-based copolymers have a density of less than 0.89g/cm³ and preferably less than 0.88 g/cm³. Both the ethylene-based copolymers and the propylene-based copolymers have a melt index of 100 g/10 min. or less and preferably 50 g/10 min. or less and most preferably 10 g/10 min. or less.

### BRIEF DESCRIPTION OF THE DRAWINGS

The drawings illustrate the best mode presently contemplated of currying out the invention.

In the drawings:

Fig. 1 is a front perspective view of a disposable diaper incorporating a first embodiment of the cling film fastener system of the present invention;

Fig. 1A is a cross-sectional view of a cling film fastener in accordance with the present invention;

Fig. 2 is a perspective view illustrating a portion of a diaper showing the initial closure made with the cling film fastener system of Fig. 1;

Fig. 3 is a cross-sectional view taken through the plane of the line 3-3 in Fig. 2;

Fig. 4 is a front perspective view of a disposable diaper incorporating a second embodiment of a cling film fastener system of the present invention;

Fig. 5 is a perspective view of a portion of a diaper showing the initial closure made with the fastener system of Fig. 4;

Fig. 6 is a cross-sectional view taken along the plane of the line 6-6 in Fig. 5;

Fig. 7 is a front perspective view of a disposable diaper incorporating a third embodiment of a cling film fastener system of the present invention;

Fig. 8 is a perspective view of a portion of a diaper showing the initial closure made with the fastener system of Fig. 7;

Fig. 9 is a cross-sectional view taken along the plane of the line 9-9 in Fig. 8;

Fig. 10 is a front perspective view of a disposable diaper incorporating a fourth embodiment of a cling film fastener system of the present invention;

Fig. 11 is a perspective view of a portion of a diaper showing the initial closure made with the fastener system of Fig. 10;

Fig. 12 is a cross-sectional view taken along the plane of the line 12-12 in Fig. 11;

Fig. 13 is a front perspective view of a disposable diaper incorporating a fifth embodiment of a cling film fastener system of the present invention;

Fig. 14 is a front perspective view of a disposable diaper incorporating a sixth embodiment of a cling film fastener system of the present invention;

Fig. 15 is a front perspective view of a disposable diaper incorporating a seventh embodiment of a cling film fastener system of the present invention;

Fig. 16 is a front perspective view of a disposable diaper incorporating an eighth embodiment of a cling film fastener system of the present invention;

Fig. 17 is a plan view of an absorbent article comprising a feminine care pad incorporating a cling film fastener system of the present invention;

Fig. 18 is a perspective view of the feminine care pad of Fig. 17 shown as it would be worn by a user;

Fig. 19 is a schematic view illustrating production of a snack food package, such as a candy wrapper, incorporating the cling film fastener system of the present invention;

Fig. 20 is a schematic perspective view of a corrugated box incorporating the cling film fastener system of the present invention on the top flaps thereof;

Fig. 21 is a plan view of an envelope incorporating the cling film laminate fastener system of the present invention;

Fig. 22 is a graph of peel strength versus density of various cling film laminate structures constructed in accordance with the present invention using a Davis Standard extruder (Example 4 data) and a Randcastle Monolayer extruder (Example 5 data);

### DETAILED DESCRIPTION OF THE INVENTION

The cling film fastener has as one of its primary components a cling layer containing one or more polyolefin copolymers that provides autoadhesive or cling surface properties. In one embodiment, the cling layer may be a substantially non-stretchable self-supporting sheet in the form of a monolayer film of the one or more polyolefin copolymers. In use, the cling film monolayer is bonded directly to a target surface so that its cling surface is exposed.

In another embodiment, the cling film fastener comprises a multi-layered laminate structure. In this embodiment, the cling layer has one or more polyolefin copolymers that provides autoadhesive or cling surface properties bonded to or coated on a carrier layer which provides support for the cling layer. The carrier layer itself may be a single layer or a multi-layered construction, and either, or both, of the cling layer and the carrier layer (or any or all of the individual layers of the carrier layer) may be substantially non-stretchable so as to render the entire cling film fastener itself substantially non-stretchable. The carrier layer preferably is the component of the laminate that eliminates, or substantially limits, stretching of the cling layer. The carrier layer thus preferably provides dimensional stability both in the longitudinal and/or the cross direction to prevent stretching or deformation of the cling layer. In use, the carrier layer is bonded directly to a target surface so that the cling surface is exposed.

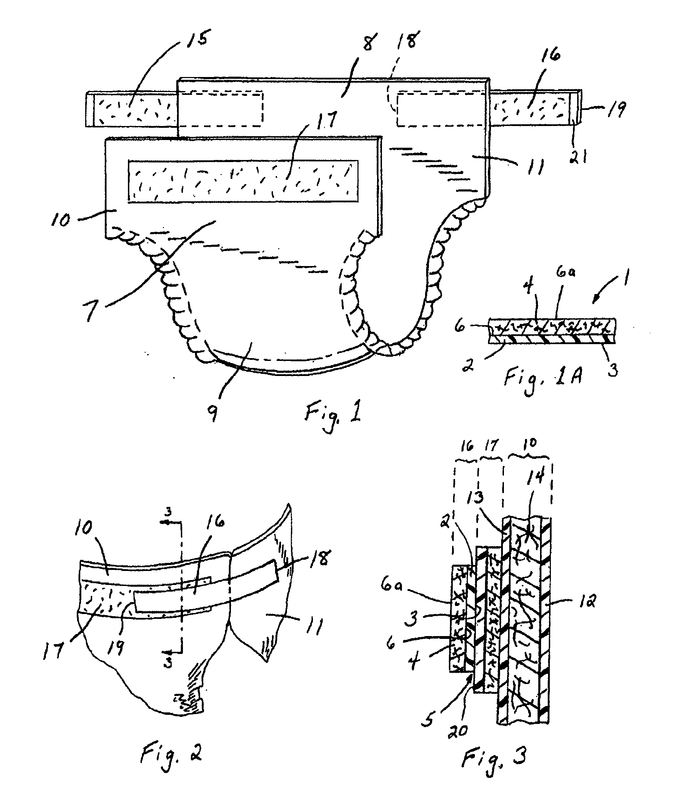

Referring now to the drawings, there is illustrated various embodiments relating to a fastening system for numerous different applications, which in the form illustrated in Figs. 1-16 comprises a multi-layered laminate including a cling layer having an outer surface with autoadhesive or cling properties at ambient temperature integrally joined and bonded to a substantially non-stretchable carter layer. The structures of the present invention are useful as various components of nonwoven soft goods, and are particularly useful in fastening systems for disposable soft goods, especially absorbent soft goods articles such as disposable diapers and feminine sanitary napkins used to absorb and contain exudates such as blood and urine discharged from a person's body, surgical drapes, hospital gowns, face masks, and hospital pads, and other articles having one or more nonwoven layers. The present fasteners are also useful in various packaging applications, such as for packaging of various food products, as previously described herein, but are not intended for use as stretch wrap material.

A laminate such as that illustrated in Figs. 1-16 is particularly adapted for use with fastening systems for disposable soft goods articles and is composed of a polyolefin copolymer cling layer and a non-stretchable carrier layer that provides a fastening system with relatively low peel, but relatively high shear strength. In such applications, the autoadhesive or cling properties of the outer surface of the cling layer eliminate or substantially reduce the contamination problems of the prior art systems using pressure sensitive adhesives. In addition, the low peel, high shear properties of the structure of the present invention eliminate or substantially reduce the deformation and deterioration problems of prior art systems using an autoadhesive film layer such as those described in Mann et al U.S. Patent 5,085,655 (block copolymer based).

The material useful for forming the carrier layer, or any of its individual layers in a multi-layered construction, is any material which is substantially non-stretchable in the machine direction (longitudinally) and/or cross machine direction (transverse). Preferably, the carrier layer is comprised of a non-woven substrate or a thermoplastic film material. By "non-woven material" it is meant a sheet or web structure bonded together by entangling fiber or filaments (and by perforating films) mechanically, thermally or chemically. They are flat, porous, self-supporting sheets that are made directly from separate fibers or from molten plastic or plastic film. They are not made by weaving or knitting and do not require converting the fibers to yarn. The carrier layer may be either flexible or non-flexible so long as it is substantially non-stretchable. The term "flexible" means that the carrier layer may be bent to a radius of 0.5 cm without breaking or cracking.

The thermoplastic film materials useful for forming the substantially non-stretchable carrier layer of the cling film fastener include meltable film-forming thermoplastics which preferably do not adhere to the cling layer's autoadhesive or cling surface at ambient temperature or service temperatures. More preferably, the thermoplastic film should have a melt temperature sufficiently close to that of the polyolefin copolymer cling layer to enable-co-extrusion of the carrier layer and the polyolefin copolymer cling layer and formation of a permanent bond therebetween, with or without the use of an adhesive therebetween, which bond is retained after cooling. In practice, any thermoplastic material may be used which is capable of being formed into a self-supporting continuous sheet or film having adequate mechanical properties to withstand normal handling and to fulfil the requirements of the end use application including satisfactory bonding with the polyolefin copolymer cling layer at an elevated temperature, and to form a substantially non-stretchable carrier layer. Thus, the carrier layer is formed of a material which eliminates, or substantially limits, stretching of the polyolefin copolymer cling layer. The carrier layer thus provides dimensional stability both in the longitudinal as well as the cross-wise or transverse direction to prevent stretching or deformation of the polyolefin copolymer cling layer. The term "substantially non-stretchable" means that preferably, during anticipated use and/or storage, the cling film fastener will stretch no more than about 50% in either its longitudinal or cross direction, and more preferably stretching should be limited to no more than 25% from its original non-stretched configuration, and most preferably less than 10% from its original non-stretched configuration. To accomplish this, one or more layers of the cling film fastener (e.g. the cling layer, the carrier layer, and/or the individual substrates or layers of the carrier layer) must be substantially non-stretchable which will thus result in the cling film fastener itself stretching during normal use no more than about 50% in either direction, and more preferably stretching no more than about 25%, and most preferably less than 10% from its original non-stretched configuration. Additionally, the carrier layer can be made breathable by any method known in the art.

The thermoplastic film material forming the carrier layer, including any of the individual layers themselves that may comprise a multi-layered carrier layer, may comprise a wide range of polymers, copolymers, terpolymers, interpolymers and blends thereof selected to meet the end use application. Illustrative thermoplastics which may be used alone or in blends include polyolefins such as polyethylene, polypropylene and polybutylene, copolymers of ethylene and C₃-C₈ olefins, thermoplastic polyesters, polyamides such as nylon, polysulfones, acrylic polymers such as polyethylene acrylic acid, polyethylene ethyl acrylate, polyethylene n-butyl acrylate and polyethylene methyl acrylate, polystyrene, polyurethanes, polycarbonates, halogenated polymers such as polyvinylchloride and polyvinylidene chloride, cellulosics, polyacrylonitriles, ethylene vinyl acetate, and ionomers based on sodium or zinc salts of ethylene/methacrylic acid. The preferred thermoplastic film materials comprise polyolefins including low, medium and high density polyethylene such as low density polyethylenes (LDPE), linear low density polyethylenes (LLDPE), high pressure low density polyethylenes (HPLDPE) and very low density polyethylenes (VLDPE), and/or polypropylene.

The cling film fastener may be a self-supporting sheet which is in the form of a monolayer or in the form of a multi-layered laminate structure. As a monolayer, the fastener comprises only the cling layer and is substantially non-stretchable, and is in the form of a self supporting sheet or film of one or more polyolefin copolymers having both its inner and outer surfaces exhibiting cling properties. It may, or may not, need to be bonded to a carrier layer to form a multi-layered laminate depending upon its end use.

The cling film fastener, however, is typically in the form of a multi-layered laminate composed of the cling layer providing autoadhesive or cling surface properties bonded to a carrier layer which in turn may be a single substrate or may also comprise a multi-layered construction. For example, the cling layer may be composed of one or more polyolefin copolymers, and the carrier layer may be comprised of a base layer (to be attached to a target surface) and a structural lawyer interposed between the base layer and the polyolefin copolymer layer. In this multi-layered construction, each of the three layers are integrally bonded together, as by coextrusion, meltblown or spunbond techniques or an adhesive, to form the desired fastener of the present invention. In addition, the cartier layer may include one or more other substrates either between the structural layer and the cling layer, or between the structural layer and the base layer. For example, depending upon the tackiness of the autoadhesive surface, the carrier layer may need a release or slip layer thereon to prevent blocking when the fastener is stored in roll form. Also, depending on its end use, the fastener may need a tear resistant substrate or layer, or a puncture resistant substrate or layer. It should also be noted that in an alternate form the cling layer that provides the autoadhesive or cling surface properties for the cling film fastener may be directly bonded (as for example by coextrusion, melt blowing or spun bonding) onto the carrier layer to form the desired laminate with or without any other layer such as the structural substrate layer. Additionally, the cling layer and/or the carrier layer can be made breathable by any method known in the art.

The terms "autoadhesive" and "autoadhesion" and "cling" are used herein to indicate the self-adhesive or cohesive-adhesive properties of a polymeric material which enable films, layers or coatings thereof to be repeatedly adhered together by application of pressure at service temperatures or room temperatures and separated. Such materials adhesively bond to each other but are substantially non-adhesive with respect to other materials. The bonds formed by the outer cling surface may be permanent, releasable, sealable, re-sealable and/or non-resealable depending upon the desired end use. The term "service temperature" is used herein in accordance with its ordinary meaning to indicate the intended temperature or temperature range of use for the cling film fastener by the end user and/or under storage conditions of the end product. Thus, the service temperature typically ranges from a temperature of about -18°C (0°F) to a shipping and storage temperature of about 60°C (140°F). The term "self-supporting" refers to the ability of a coating, layer or film of material to independently support itself or its own weight.

The terms "fasten", "fastening", or "fastener" as used herein is intended to broadly refer to the attaching of one item to another whether the resultant attachment is permanent, releasable, sealable, re-sealable, non-resealable, reclosable, or the like. As such, these terms are intended to cover applications where cold seals, pressure sensitive adhesives, hot melt adhesives and/or curable adhesives have been used to bond two items together in the past, but is clearly not limited to such conventional adhesive bonding techniques or applications.

The individual layers of the carrier layer may be composed of a thermoplastic material an may be comprised of any suitable polyolefin or combination of polyolefins such as polyethylene, polypropylene, copolymers of ethylene, copolymers of propylene, or polymers obtained from ethylene and/or propylene copolymerized with other olefins, particularly C₃ to C₁₂ olefins. Especially preferred olefins are 1-butene, 1-hexene, 1-octene and 4-methyl pentene-1. Particularly preferred materials for use as one or more of the individual layers of the carrier layer are low, medium and/or high density polyethylene, polypropylene, and ethylenic copolymers such as linear low density polyethylenes (LLDPE) or very low density polyethylenes (VLDPE).

Suitable ethylene-based copolymers for use as the individual layers of the carrier layer comprise a major proportion by weight of ethylene copolymerized with a minor proportion by weight of an alpha-olefin monomer containing about 3 to about 12, preferably about 4 to about 10, and more preferably about 4 to about 8, carbon atoms. These resins have a polydispersity which is preferably in the range of from about 2 to about 7.

Ethylene-based copolymers for use as the individual layers of the carrier layer are those commonly referred to as linear low density polyethylenes (LLDPE) and very low density polyethylenes (VLDPE). Preferably the ethylene-based copolymers employed are those having from about 1 to about 20, preferably from about 1 to about 10 weight percent of said higher alpha-olefin monomer copolymerized therein. In addition, the alpha-olefin monomer employed in the ethylene-based copolymer is selected from the group consisting of 1-butene, 3-methyl-1-butene, 3-methyl-1-pentene, 1-hexene, 4-methyl-1-pentene, 3-methyl-1-hexene, 1-octene and 1-decene. Particularly preferred are the 1-butene, 1-octene and 1-hexene alpha-olefins. The LLDPE resins may be prepared at relatively low pressures employing coordination-type catalysts. Reference may be made to U.S. Patent Nos. 3,645,992, 4,076,698, 4,011,382, 4,163,831, 4,205,021, 4,302,565, 4,302,566, 4,359,561 and 4,522,987 for more details of the manufacture and properties of LLDPE resins including those which are particularly useful herein.

The LLDPE resins that can be used in the carrier layer herein have a density ranging from about 0.90 to about 0.940g/cm³, more commonly from about 0.90 to about 0.93g/cm³, and a melt index (I₂) of from about 0.5g/10 min. to about 30g/10 min., preferably from about 1 to about 10g/10 min., as determined by ASTM D1238. Particularly preferred are those LLDPE resins possessing densities within the range from about 0.917 to about 0.920gm/cm³ and a melt index of from about 2.0 to about 5.0g/10 min., as determined by ASTM D1238. Examples of such LLDPE resins include those set forth in U.S. Patent No. 5,273,809, which is incorporated herein by reference in its entirety. Such LLDPEs and methods for making them are well known in the art and are readily available commercially.

The VLDPE resins that may be used in the carrier layer herein have a density ranging from about 0.880 to about 0.912g/cm³, more commonly from about 0.89 to about 0.91g/cm³, and a melt index of from about 0.5 to about 5g/10 min., preferably from about 1 to about 3g/10 min. Such VLDPEs and methods for making them are well known in the art and are readily available commercially.

Suitable polypropylene is normally solid and isotactic having a wide ranging melt index between 0.1g/10 min. to 300g/10 min. Such polypropylene is normally crystalline with a density range of from about 0.89g/cm³ to about 0.91g/cm³ for isotactic polypropylene. Such polypropylenes and methods for making them are well known in the art and are readily available commercially.

The thermoplastic materials useful in the carrier layer, and particularly, the LLDPE and VLDPE resins, can be blended with minor amounts, e.g., up to about 40 weight percent total, of one or more other suitable resins to achieve a desired range of physical/mechanical properties in the film product. Thus, for example, such resins as ethyl vinyl acetate (EVA) copolymer, high pressure low density polyethylene (HPLDPE), and other LLDPE resins may be used for blending to obtain useful mixtures for forming one or more layer of the carrier layer of the present invention.

The thermoplastic polymer material providing the autoadhesive or cling surface properties for the cling layer may be composed of a thermoplastic material or blends of thermoplastic materials which are selected from the group consisting of polyolefins, acrylic modified polyolefins, vinyl acetate modified polyolefins, and acrylic polymers. The polyolefin may be polypropylene or polyethylene. The acrylic modified polyolefin may be a copolymer of polypropylene or polyethylene and an acrylic. Likewise, the vinyl acetate modified polyolefin may be a copolymer of polypropylene or polyethylene and vinyl acetate.

The thermoplastic polymer material that provides the autoadhesive or cling properties for the cling layer is preferably comprised of a suitable single site or metallocene catalyzed ethylene-based copolymer comprising a major portion by weight of ethylene and a minor portion by weight of a C₃ to C₁₈ alpha-olefin comonomer, or a single site or metallocene catalyzed propylene-based copolymer comprising a major portion by weight of propylene and a minor portion by weight of a C₂ to C₁₈ alpha-olefin comonomer, or a blend of the ethylene-based copolymers, the propylene-based copolymers, or one or more of the ethylene-based copolymers with one or more of the propylene-based copolymers. The alpha-olefin comonomer preferably contains 3 to 12 carbon atoms, more preferably contains 4 to 10 carbon atoms, and most preferably contains 4 to 8 carbon atoms. More particularly, the alpha-olefin comonomer may be selected from 1-butene, 1-pentene, 3-methyl-1-butene, 3-methyl-1-pentene, 1-hexene, 4-methyl-1-pentene, 1-dodecene, 3-methyl-1-hexene, 1-octene, and 1-decene. Particularly preferred is 1-octene copolymerized with ethylene.

The alpha-olefin comonomer content in the ethylene-based copolymer is at least 20% by weight and in the range of from 20% to 50% by weight, preferably from 25% to 50% by weight, more preferably from 30% to 50% by weight. Suitable ethylene-based copolymers have a density as determined by ASTM D-792 of 0.89g/cm³ or less and in the range of from 0.89g/cm³ to 0.85g/cm³, preferably between 0.88g/cm³ and 0.85g/cm³, and most preferably between 0.875g/cm³ and 0.85g/cm³. Suitable ethylene-based copolymers also have a melt index at 190°C under 2.16 kg as determined by ASTM D 123 8 of 100 g/10 min. or less, preferably 50 g/10 min. or less, more preferably 10 g/10 min. or less, and most preferably 5 g/10 min. or less.

The alpha-olefin comonomer content in the propylene-based copolymer is at least 5%, preferably 5% to 30%, and most preferably 5% to 15% by weight, and the preferred copolymer is a propylene-ethylene copolymer. The propylene-based copolymers have a melt index (measured at 230°C) of less than 100 g/10 min., preferably less than 50 g/10 min. and more preferably less than 25 g/10 main.

"Blends" may comprise two or more ethylene-based copolymers or two or more propylene-based copolymers, or one or more ethylene-based copolymers with one or more propylene-based copolymers. Where a blend of copolymers is used, the calculated density of the blend should also fall under the above limits, i.e., less than 0.89 g/cm³. For example, a blend of 70% of an ethylene-based copolymer having a density of 0.870 g/cm³ and 30% of a propylene-based copolymer having a density of 0.885 g/cm³ will result in a final blend having a calculated density of 0.875 g/cm³.

Useful single site or metallocene catalyzed ethylene-based polymers are available from, among others, Dow Chemical Company and Exxon Mobil Chemical Company who are producers of single site or constrained geometry catalyzed polyethylenes. These resins are commercially available as the AFFINITY and EXACT polyethylenes (see Plastics World, pp. 33-36, January 1995), and also as the ENHANCED polyethylene and EXCEED line of resins. These ethylene-based copolymers are also available under the ENGAGE brand from DuPont Dow Elastomers. The manufacture of such polyethylenes, generally by way of employing a metallocene catalyst system, is set forth in, among others, U.S. Patent Nos. 5,382,631,5,380,810,5,358,792,5,206,075,5,183,867, 5,124,418, 5,084,534, 5,079,205, 5,032,652, 5,026,798, 5,017,655, 5,006,500, 5,001,205, 4,937,301, 4,925,821, 4,871,523, 4,871,705 and 4,808,561, each of which is incorporated herein by reference in its entirety. These catalyst systems and their use to prepare such copolymer materials are also set forth in EP0600425A1 and PCT applications WO 94/26271 and WO 94/26816.

The single site or metallocene catalyzed propylene-based copolymers are available under the VERSIFY brand from The Dow Chemical Company. The manufacture of such polypropylenes is also based on using a metallocene or single site catalyst system and is based on Dow's INSITE technology.

The thermoplastic polymer coatings used to provide the autoadhesive or cling properties may also contain known and conventional cling additives to augment the cling property that, at least in the case of the particularly preferred resins, is inherently exhibited. Examples of useful cling additives include polyisobutylenes having a number average molecular weight in the range of from about 1,000 to about 3,000, preferably about 1,200 to about 1,800, was measured by vapor phase osmometry, amorphous atactic polypropylenes, e.g., those having an average molecular weight of about 2000, and polyterpenes and ethylene-vinyl acetate copolymers containing about 3 to about 90 weight percent copolymerized vinyl acetate. The optional cling additive can be present in a concentration of from-about 0.5 to about 10 weight percent of the resin.

Additionally, small amounts, less than 25% by weight and more preferably less than 10% by weight of modifiers can be added to modify the autoadhesive or other characteristics of the cling layer. Examples of these include tackifying resins, plasticizers, waxes, fillers, antioxidants, colorants, antiblocking agents, antistatic agents, UV stabilizers, etc.

Additionally, the cling surface may be modified to improve and/or change the blocking or cling properties, peel strength and/or shear strength in other ways. This may be accomplished by mechanical means (e.g. embossing or stamping techniques) or via the use of energy (e.g. UV, RF, microwaves or heat).

The thermoplastic polymer coatings used to provide the autoadhesive or cling properties may also be treated to such known and conventional post-forming operations as corona discharge, chemical treatment, flame treatment, etc., to modify the printability or ink receptivity of the surface (s) or to impart other desirable characteristics thereto. Thus, the fastening structure of the present invention may be pigmented, transparent, opaque or contain printing on selected portions thereof.

The thermoplastic polymer coatings used to provide the autoadhesive or cling properties is preferably constructed entirely from either an ethylene-based copolymer or propylene-based copolymer produced by single site or metallocene catalyst technology as defined herein. However, the thermoplastic polymer coating may also comprise a blend of one or more of the metallocene catalyzed copolymers with a second resin material. The second material may be an olefin polymer resin such as a polyolefin like polypropylene or polyethylene, an acrylic modified polyolefin, a vinyl acetate modified polyolefin or an acrylic polymer. For example, this may include, but is not limited to, LLDPE, LDPE, HPLDPE, VLDPE, propylene based resins or combinations thereof. If the second resin material is to be incorporated with the metallocene or single site-catalyzed resin, it is preferred to maintain the level of the metallocene-catalyzed resin to at least about 60wt. %. The resultant blended polymer mixture maintains the desired properties of the metallocene-catalyzed material and may be more economical for certain applications.

The thermoplastic polymer coatings may be directly coextruded onto or bonded with an adhesive to a structural substrate to form the cling layer in any conventional manner. Alternately, if it was desired to eliminate the structural substrate of the cling layer, the polymer coating could be extruded directly onto the base carrier layer or could be coextruded along with the base carrier layer or be meltblown or spunbond directly onto the base carrier layer.

In one embodiment, and for example when used in a nonwoven soft goods application (e. g. Figs. 1-16), the autoadhesive surface of the cling layer provides an adhesive surface that has relatively low peel strength, but relatively high shear strength. By "low" peel strength, it is meant that the peel strength of the cling layer is preferably 39 kg/m (1000g/inch) or less, more preferably 24 kg/m (600g/inch) or less, and most preferably 16 kg/m (400g/ich) or less as determined by the peel test method described herein in Example 1. By "high" shear strength, it is meant that the shear strength of the cling layer is preferably greater than 4 hours, and most preferably greater than 8 hours as determined by the shear strength test hereinafter described. Thus, the low peel, but high shear strengths of the cling layer's autoadhesive surface provide sufficient peel and shear forces to hold the diaper in place yet enables the fastening tab of a disposable diaper to be readily opened by a user without rupturing or significantly damaging the front panel of the disposable diaper while at the same time allowing the tabs to be refastened if desired. Three examples of cling films that may be useful in the present laminate can be found in U. S. Patent No. 5, 049, 423, U. S. Patent No. 5,085, 927 and U. S. Patent No. 5,902, 684. Other examples of cling film can be found in U. S. Patents 5,093, 188 and 5,208, 096. The preferred cling film useful in the present laminate is a polyethylene film available under the trade name "Presto" from Presto Products Co. of Appleton, Wisconsin. Two grades particularly well suited are Presto CNC10152 and101515. Another preferred polyethylene cling film is available under the trade name "Paragon" from Paragon Films, Inc. of Broken Arrow, Oklahoma. Examples from Paragon include V109015A, T128370 Global and T817125. Yet another preferred cling film is an ethylene-alkyl acrylate available under the tradename "Pactiv APM3-2015" from Pactiv Corporation of Lake Forest, Illinois. "Masking" films are also available from Tredegar Co.

The fastener of the present invention may be prepared by extrusion processing of the cling layer directly onto the carrier layer using any conventional commercially available apparatus. Alternately, the cling layer may be adhesively bonded to the carrier layer, or it may be meltblown or spunbond thereon. Any other bonding method may be used to bond the cling film layer to the carrier layer, e. g. ultrasonic, thermal, pressure bonding, microwave, RF, etc. In addition, the carrier layer could be directly formed onto the cling layer. For example, if the carrier layer is a nonwoven material, the nonwoven can be directly melt blown or spun bonded onto the cling layer. If an adhesive is used to bond the layers of the fastener together, the adhesive may be any suitable hot melt adhesive, and may be applied using any standard application equipment either to the cling layer or to the carrier layer or to both. Typical add-on levels for the adhesive layer would be from about1 g/sq. meter to about 20g/sq. meter. It should be noted that the particular apparatus selected, whether it be a co-extrusion apparatus or a coater/lamination apparatus, may depend upon the differences in processing temperatures and rheologies of the materials forming the cling layer, the carrier layer, and the optional adhesive layer. The overall thickness of the fastener or laminate can vary widely and is application specific. Generally, however, the laminate will be 5µm to 1mm (0.2 mils to 40 mils) thick, and typically ranging between 13µm and 0.5 mm (0.5 and 20 mils) in thickness.

Target surfaces are those surfaces the fastener or cling film laminate of the present invention is bonded to. Target surfaces which are useful in the invention may be selected from a wide variety of materials. Examples of useful target surfaces include the surfaces of those materials previously identified as being useful for the individual layers of the carrier layer as well as polymeric materials such as polycarbonate, polyacrylonitrile, butadiene-styrene polymers, poly (methylmethacrylate), polyamide, ethylene vinylacetate copolymer, treated and untreated poly (ethylene terephthalate), Surlyng, polystyrene, acrylonitrile butadiene-styrene polymer, polypropylene, and polystyrene. Target surfaces also include metallic surfaces such as stainless steel; glass; paper of all types including cardboard, paperboard and coated paper stock; enamel coated substrates; and particularly nonwoven surfaces. The fastener or cling film laminate of the present invention may be bonded in any manner to the target surface. For example, the bond may comprise a pressure sensitive adhesive coated on the carrier layer or the target surface, a hook and loop reclosable fastener, a mushroom-shaped reclosable fastener, an ultrasonic weld, a mechanical bond, or a thermal bond. The exact choice of target surface and bonding method to be used is dependent upon the needs of the user.

Referring now to Figs. 1-3, and particularly to Fig. 1A, a laminate 1 of multi-layer construction includes a cling layer 2 having an autoadhesive surface 3 bonded to and integrally joined with a flexible, but substantially non-stretchable, carrier layer 4. The cling layer 2 and carrier layer 4 are bonded together along an interface 6 formed by the adjacent interior surfaces of layer 2 and layer 4. As illustrated, cling layer 2 is co-extruded directly onto carrier layer 4. Both the cling layer 2 and the carrier layer 4 may range in thickness from about 2.5µm to 500µm (0.1 mil to about 20 mils).

The cling layer 2 is formed of a suitable thermoplastic polymer material, such as polyethylene. The exterior surface 3 of layer 2 is autoadhesive in that it mutually adheres to like autoadhesive surfaces, but is otherwise substantially non-adhesive. The carrier layer 4 is formed of a non-woven material and includes exterior surface 6a which does not substantially adhere to surface 3 when they are pressed together at room temperature or elevated storage temperature conditions, even when wound under tension in large diameter rolls. Accordingly, the laminate 1 may be self-wound or stacked without a release liner thus enabling the laminate to be manufactured in the form of a web and stored in the form of a roll for use in conventional diaper manufacturing systems.

As shown in Figs. 1-3, laminate 1 is illustrated as being a fastener component useful in a fastening system for a disposable diaper. As illustrated, the disposable diaper generally comprises a front panel 7 and a rear panel 8 joined together by a crotch section 9. The front panel 7 and rear panel 8 each have waist portions 10 and 11 respectively and encircle an infant's body, and are overlapped and joined together by the diaper fastening system to hold the diaper in place. The disposable diaper itself comprises a three-layer composite structure including a liquid permeable body side inner liner or top sheet 12, a liquid impermeable outer layer or back sheet 13, and a batt or core 14 of absorbent material sandwiched between the inner liner 12 and outer cover 13. As illustrated, a pair of diaper fastening tabs 15 and 16 each incorporating laminate 1 as its principal component is illustrated. Laminate 1 may be sold in roll form to a diaper manufacturer for die cutting to form tabs 15 and 16. The tabs 15 and 16 are secured to the outer liner 13 of the disposable diaper in a conventional construction.

As further illustrated in Figs. 1-3, a fastening tab landing zone 17 incorporating laminate 1 as its principal component is secured to outer liner 13 along front panel 7. Landing zone 17 is conventionally adhesively bonded to the outer surface of cover 13, although other bonding means can be used. An additional piece of laminate (not shown) can be placed in a separate area to facilitate closure after the diaper is soiled for disposal purposes, if desired.

As shown best in Fig. 1, tabs 15 and 16 each has an elongate rectangular shape including an inner end 18 attached to waist portion 11 of rear panel 8 and an outer end 19. The carrier layer 4 of laminate 1 at the terminal edge of outer end 19 extends slightly beyond the cling layer 2 at the terminal edge of outer end 19 in the storage position and during use of the diaper to provide a finger lift area 21. No release liner or protective tab is required along the finger lift portion of outer end 19 since autoadhesive surface 3 is substantially non-adhesive and non-tacky. For purposes of diaper closure, the autoadhesive surface 3 of outer end 19 of fastening tabs 15 and 16 are pressed against the autoadhesive surface 20 of landing zone 17 forming a cling-to-cling interface 5. As noted earlier, tabs 15 and 16, as well as landing zone 17 is formed using laminate 1, and the overall configuration is illustrated in section in Fig. 3. Thus, the autoadhesive surface 3 of fastening tabs 15 and 16 are pressed against the autoadhesive surface 20 of landing zone 17. Thus, tabs 15 and 16 may be "fastened" or attached to landing zone 17 resulting in waist portions 10 and 11 joined together to hold the diaper in place.

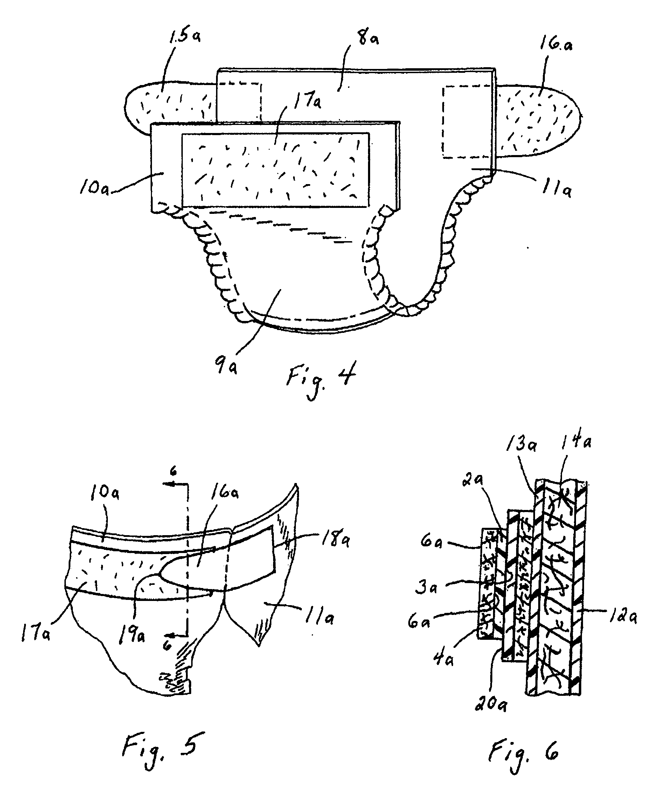

Figs. 4-6 illustrate a disposable diaper incorporating a second embodiment of the cling film fastening system of the present invention. In this second embodiment, like components are numbered similarly as the first embodiment except using the subscript "a". As illustrated, the only significant difference between this second embodiment of a diaper fastening system and the first embodiment shown in Figs. 1-3, is that landing zone 17a is slightly larger than landing zone 17 shown in Fig. 1, and fastening tabs 15a and 16a are in the shape of ears instead of conventional rectangular shaped tapes. In all other aspects, the second embodiment of Figs. 4-6 is the same as the first embodiment of Figs. 1-3.

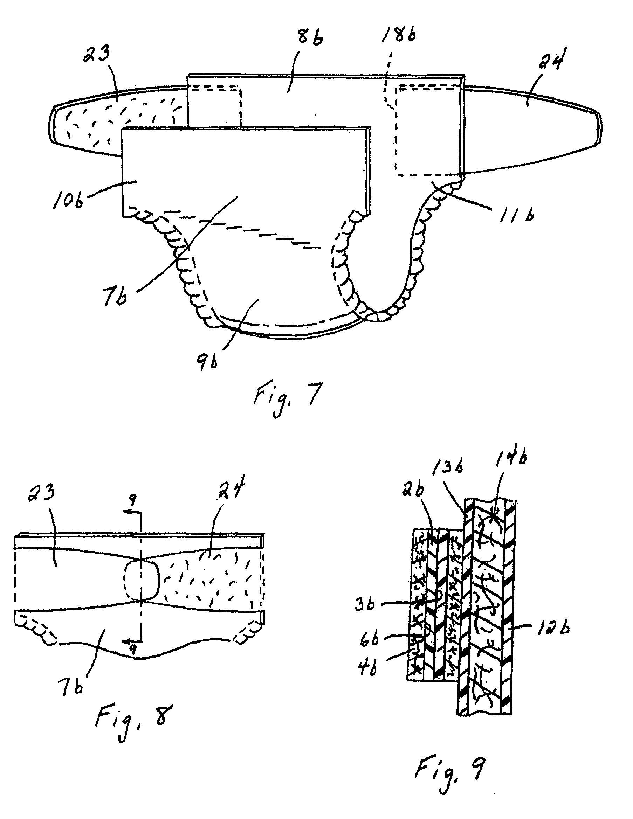

Referring now to Figs. 7-9, there is illustrated a disposable diaper incorporating a third embodiment of the cling film fastening system of the present invention. In this third embodiment, like components are numbered similarly as the first and second embodiments except using the subscript "b". This third embodiment eliminates the use of a landing zone on the front panel of the diaper. Instead, it incorporates a pair of large ears 23 and 24 with the autoadhesive surface of the cling layer on one ear 23 facing up and the autoadhesive surface of the cling layer of ear 24 facing down such that the outer edges of ears 23 and 24 overlap to provide a diaper fastening or closure system.

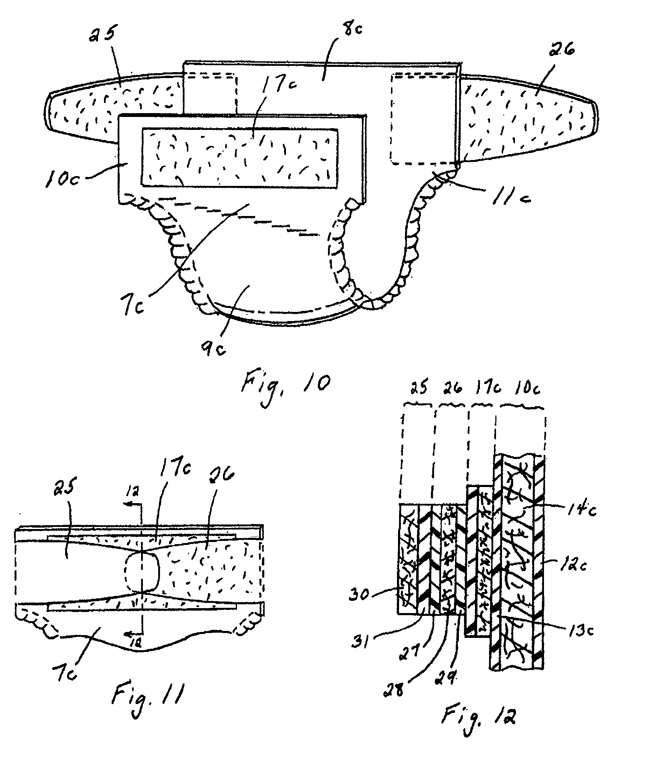

Figs. 10-12 illustrate a disposable diaper incorporating a fourth embodiment of the cling film fastening system of the present invention. In this fourth embodiment, like components are numbered similarly as the first through third embodiments except utilizing the subscript "c". This fourth embodiment is similar to the third embodiment of Figs. 7-9 except it utilizes a landing zone 17c on the front panel 7c of the diaper and a pair of large ears 25 and 26 both composed of laminate 1. However, in this fourth embodiment, ear 26 has an autoadhesive surface on both sides thereof and thus forms a tri-laminate comprised of a cling layer 27, a carrier layer 28 and a second cling layer 29 as shown best in Fig. 12. Ear 25, however, is similar to ears 15a and 16a in that it has a carrier layer 30 and a cling layer 31 on only one side thereof. As a result, when the fastening system is closed, the inner ear 26 clings to landing zone 17c and the outer ear 25 clings to a portion of landing zone 17c as well as a portion of the outer edge of ear 26.

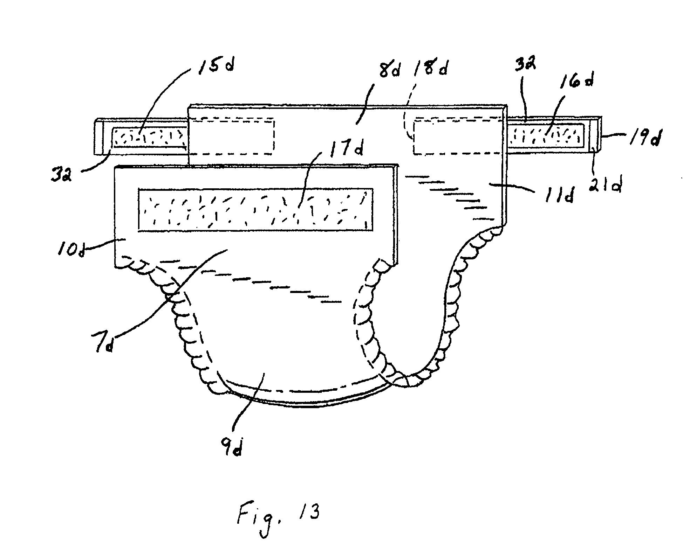

. Referring now to Fig. 13, there is illustrated a disposable diaper incorporating a fifth embodiment of the cling film fastening system of the present invention. In this fifth embodiment, like components are numbered similarly as the first through fourth embodiments except utilizing the subscript "d". This fifth embodiment is similar to the first embodiment except it utilizes an area 32 along the edges of fastening tabs 15d and 16d which contains a pressure-sensitive adhesive coated thereon. The pressure-sensitive adhesive area 32 may be utilized as an auxiliary, supplemental or secondary closure feature to ensure that the edge margins of tabs 15d and 16d are affixed to landing zone 17d. The pressure-sensitive adhesive area 32 thus ensures that the edges of tabs 15d and 16d do not curl up during use. Any pressure-sensitive adhesive commonly used in the prior art can be utilized to coat area 32, if desired. It should also be noted that areas 15d, 16d and 32 could be reversed, i.e. cling layers 15d and 16d could instead be a coating of pressure sensitive adhesive and areas 32 could be the cling layer. Thus, the primary and auxiliary fastener systems for the diaper of Fig. 13 could take on either form.

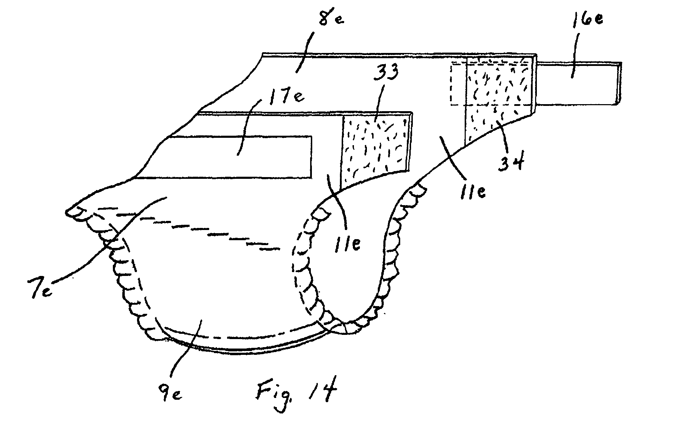

Fig. 14 illustrates a disposable diaper incorporating a sixth embodiment of the cling film fastening system of the present invention. In this sixth embodiment, like components are numbered similarly as the first through fifth embodiments except utilizing the subscript "e". This sixth embodiment utilizes a landing zone 17e on the front panel 7e of the diaper and a fastening tab 16e coated with pressure-sensitive adhesive or Velcro or other mechanical fastener. However, in the embodiment shown in Fig. 14, the waist portions 10e (not shown) and 11e of the diaper include ears 33 and 34 bonded along the sides thereof. The ears 33 and 34 are composed of the cling layer and/or laminate described herein. In use, the outwardly facing cling surface of ear 33 overlaps the inwardly facing cling surface of ear 34 to provide an auxiliary fastener or closure system, which forms a side seam for the diaper, and the tab 16e engages landing zone 17e to provide a primary fastener system for a disposable diaper. Again, it should be noted that the primary and auxiliary fastener systems could be reversed, if desired, i.e. ear 33 could be coated with pressure sensitive adhesive and tab 16e could be the cling layer/laminate.

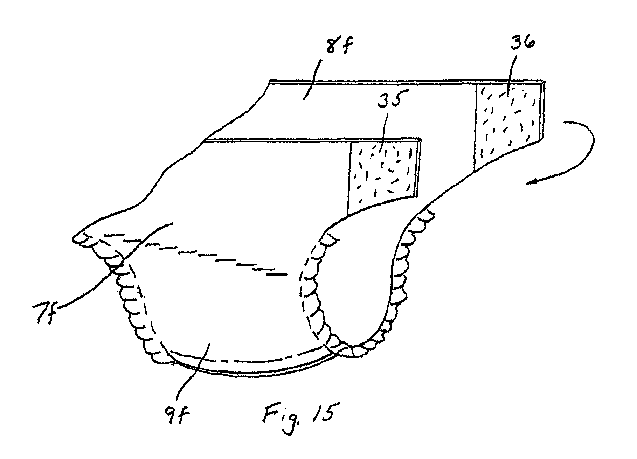

Fig. 15 illustrates a disposable diaper incorporating a seventh embodiment of the cling film fastening system of the present invention. In this seventh embodiment, like components are numbered similarly as the first through sixth embodiments except using the subscript "f". This seventh embodiment is similar to the sixth embodiment of Fig. 14 except it eliminates the fastening tab 16e and landing zone 17e shown in Fig. 14. Instead, this embodiment incorporates only the integral ears 35 and 36 to function as the fastening system for a disposable diaper. As illustrated, the autoadhesive surface of the cling layer of ear 35 faces outwardly while the autoadhesive surface of ear 36 faces inwardly so that when overlapped, the ears 35 and 36 provide a diaper fastening or closure system.

It should be noted that instead of adhesively attaching ears 35 and 36 to waist portions 10f and 11f of the diaper, ears 35 and 36 could also be formed integrally as part of the back sheet or outer liner 12f for the disposable diaper. In other words, Fig. 3 illustrates the diaper as including an inner liner or top sheet 12, an outer liner or back sheet 13, and an inner batt or absorbent core 14 sandwiched therebetween. It is contemplated that laminate 1 could replace the outer liner or back sheet 13 in such a manner that the non-woven carrier layer would face outwardly and the cling layer would face inwardly. In this manner, ears 35 and 36 could be formed integrally as part of that outer laminate forming the back sheet. Thus, in the embodiment shown in Fig. 15, the ears 35 and 36 could be integrally formed with laminate 1 as a replacement for back sheet 12 instead of adhesively attached to the sides of waist portions 10f and 11f.

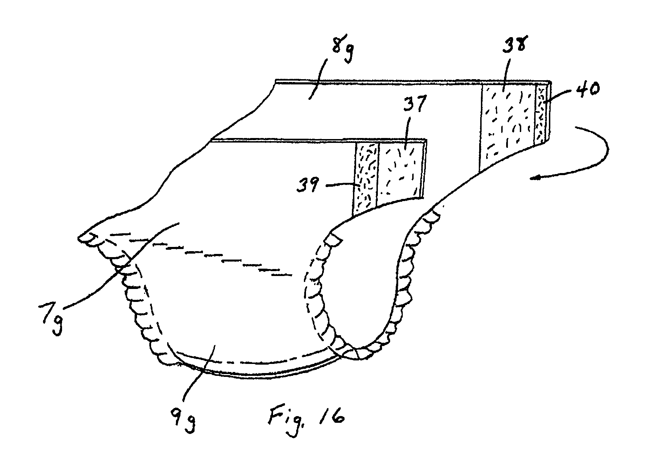

Fig. 16 illustrates a disposable diaper incorporating an eighth embodiment of the cling film fastening system of the present invention. In this eighth embodiment, like components are numbered similarly as the first through seventh embodiments except utilizing the subscript "g". This eighth embodiment is similar to the seventh embodiment except it utilizes a hook and loop fastening system as a supplement to the cling film/non-woven laminate. As illustrated, this eighth embodiment includes a pair of ears 37 and 38 wherein ear 37 has an autoadhesive surface facing outwardly and ear 38 has an autoadhesive surface facing inwardly. However, in addition, ear 37 includes a strip 39 of hook fastening material and ear 38 includes a strip 40 of loop fastening material. Thus, when ears 37 and 38 are positioned in overlapping relation, the autoadhesive surfaces cling to one another to form a primary closure system and the strips 39 and 40 provide an auxiliary or supplemental closure to ensure a more secure diaper fastening system. It should be noted that the fastening material of strips 39 and 40 could be reversed, i.e. strip 40 could be the hook fastener while strip 39 could be the loop fastener, if desired. Also, the specific location and/or configuration of strips 39 and 40 could vary depending upon the desired amount of supplemental closure desired.

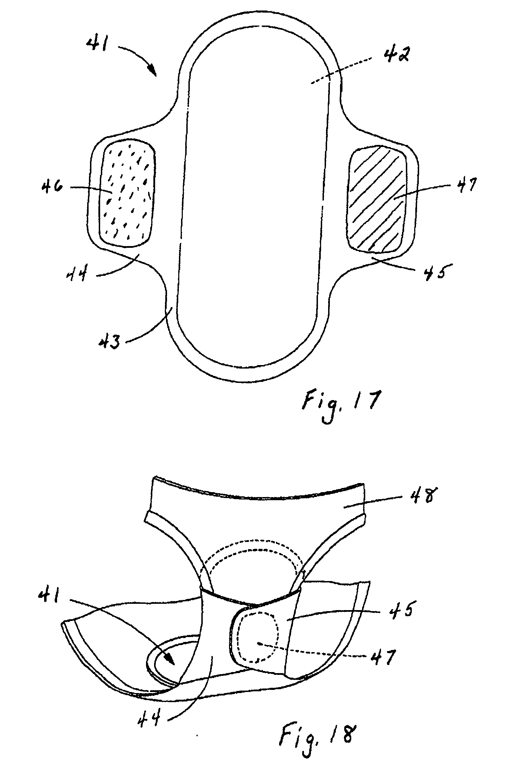

In a further alternative embodiment, the cling film laminate may serve as a fastening system for a feminine care pad or sanitary napkin 41 as shown in Figs. 17-18. The sanitary napkin 41 has a top sheet 42, a back sheet 43 and wings 44 and 45. As shown, the cling film laminate may form the back sheet 43, the wings 44, 45, or both. Preferably, the cling film laminate is disposed on wings 44 and 45 as illustrated by areas 46 and 47 respectively. Alternately, the entire wing 44 and/or 45 could be composed of the cling film laminates. It should be noted that the autoadhesive surface of area 46 faces upwardly in Fig. 17 while the autoadhesive surface of area 47 faces downwardly into the paper (as illustrated by the cross hatching) in Fig. 17. As illustrated in Fig. 18, the cling film laminates 46, 47 act to connect the wings 44 and 45 to each other around the wearer's underwear 48.

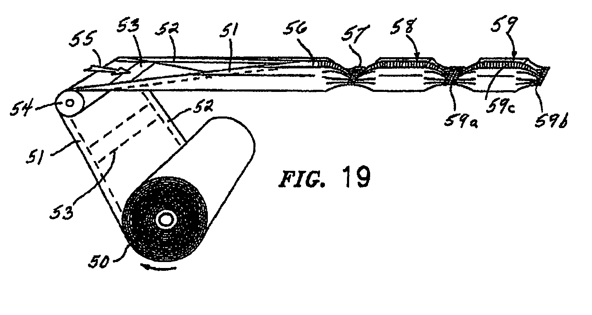

As shown in Fig. 19, flexible packaging material comprising a wrapper 50 which has already been provided with cling layers 51, 52 extending longitudinally along opposite edges thereof and spaced transversely extending cling layers 53, is rolled from a stock roll and fed around an idler roll 54 to a packaging station. It should be noted that although the cling layers and/or laminates are illustrated as being bonded to only certain portions of wrapper 50 (e.g. its opposite edges), the cling layer could also cover the entire surface of wrapper 50 if desired. Arrow 55 indicates the insertion of an article such as a candy bar or the like which is accompanied by continuously creating a longitudinal seal 56 and a transverse seal 57 by pressing the cling layers 51, 52 and 53 together to create a packaging unit 58 in any conventional manner known in this art. A cutting device (not shown) separates the unit 58 in any conventional manner by cutting along transverse seal 57 to form an individual packaged piece 59 with opposite transverse end seams 59a and 59b and a longitudinal seam 59c. It is contemplated that the cling film layer/laminate fastener could be used in any application where cold seal or cohesive coatings are currently being used, especially flexible packaging applications. In addition, it could be a substitute for mechanical attachments such as hook and loop fasteners, mushroom-shaped fasteners and "Zip Lock" seals on plastic bags..

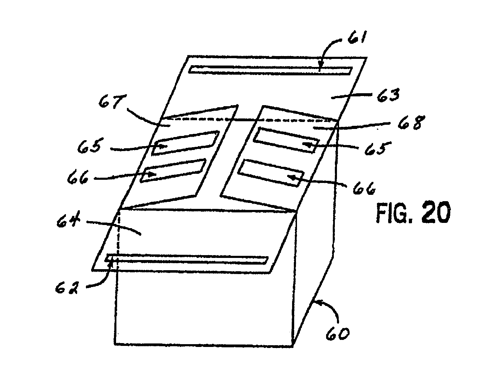

Fig. 20 illustrates a corrugated box 60 incorporating the cling film layer/laminate fastening system of the present invention. Cling layers 61,62 are applied to lower surfaces of top flaps 63,64 respectively and cling layers 65,66 are applied to the upper surfaces of top flaps 67, 68 respectively. When top flaps 63, 64 of box 60 are folded over and brought into contact with pressure against flaps 67,68 the cling layers 61,62 engage against cling layers 65,66 to seal box 60.

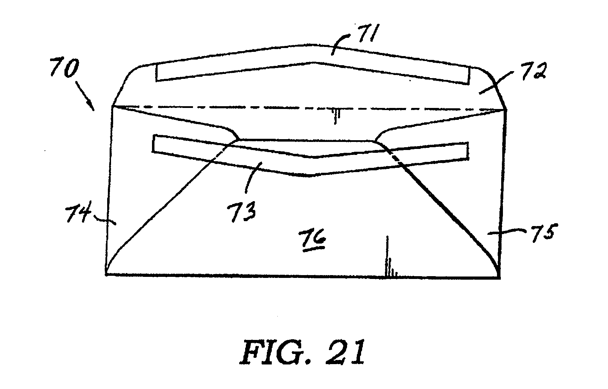

Fig. 21 illustrates an envelope 70 incorporating the cling film layer/laminate fastening system of the present invention. Cling layer 71 is applied along the inner surface edge of flap 72, and a cling layer 73 is applied long an exterior surface of an upper edge margin adjacent the opening leading to the interior of envelope 70 of side flaps 74,75 and back panel 76. When flap 72 is folded over and pressure applied thereto cling layer 71 engages against cling layer 73 to seal envelope 70 in a self-sealing manner without the need for moistening layers 71 or 73. Again, it is contemplated that the cling film layer/laminate fastener could be used in any application where pressure sensitive adhesives are currently being used.

A further embodiment of the invention includes a reclosable fastening system for use in connection with flexible packaging applications. The plastic bag is of the resealable type and is illustrated as having a generally rectangular configuration including a transparent flexible front wall or panel and back wall or panel 80. The front and back walls are made of a polymeric material which enables the bottom and side edges of the walls and to be heat sealed together to form a closed container. Suitable materials from which the walls may be formed include polyolefins such as polyethylene, polypropylene, ethylene-based copolymers and propylene-based copolymers.

In this embodiment, the back or rear wall has a height slightly greater than the height of the front wall to define an upper edge which cooperates with an upper edge of the front wall to determine an access opening into the bag when the upper edges are separated. In accordance with the present invention, a cling film fastener is applied along the inner surface of an upper edge having its cling surface exposed and directed toward the front wall. In addition, a second cling film fastener is applied along the upper edge of the front wall with its cling surface exposed and extending toward a rear wall. Thus, when pressure is applied to the upper edges so that the cling surface of the fastener engages the cling surface of the second fastener, the two opposing cling surfaces adhere to one another to close the opening. Both fasteners and could be in the form of a cling film monolayer or a cling film laminate, as described herein.

The cling film layer/laminate fastening system of the present invention is in the form of a tape. The tape is a multi-layered laminate structure and includes a cling layer and a carrier layer comprised of a structural substrate or layer and a base layer. Either or both of the carrier layers or the base layer may be substantially non-stretchable, and in addition, the layers may be specifically designed to provide desirable characteristics to the tape. For example, the carrier layer may be a tear resistant layer or may be a puncture resistance layer, or the like. In addition, the carrier layer or base layer may be formed of any suitable material, especially from plastic polymeric films such as polyolefins, as previously described herein. Layers the cling layer, carrier layer or base layer are integrally cojoined to form the tape in any manner as previously described herein. Finally, the tape includes a bonding layer formed on the base layer. The bonding layer may be any pressure sensitive adhesive commonly employed with tape to accomplish fastening of the tape to a target surface.

### EXAMPLE 1

The improved peel and shear properties of laminates made in accordance with the present invention are illustrated by the data reported in Table 1 below. For each laminate tested the peel strength was 50g or less and shear strength was acceptable (all samples held for at least 8 hours), not only at room temperature but also after aging at elevated temperatures.

**Table 1**

| | | Initial Test | | Stored 24 Hours at 120°F | | Stored 24 hours at 100°F | | |
|---|---|---|---|---|---|---|---|---|
| Cling Film | NW | Room Temp. Peel Strength (gm) | 37.8°C (100°F) Shear | Room Temp. Peel Strength (gm) | 37.8°C (100°F) Shear | Room Temp. Peel Strength (gm) | 37.8°C (100°F) Shear | Chemistry of Cling Surface |
| Pactiv APM3-2015 | Avgol | 50 | pass | 58 | pass | 34 | pass | Poly(Ethylene-alkyl acrylate) |
| Presto CNC 101515 | Avgol | 30 | pass | 22 | pass | 18 | pass | Polyethylene |
| Presto CNC 10152 | Avgol | 13 | pass | 20 | pass | 19 | pass | Polyethylene |
| Paragon V1 09015A | Avgol | 32 | pass | 19 | pass | 14 | pass | Polyethylene |
| Paragon T1 28370 Global | Avgol | 17 | pass | 24 | pass | 22 | pass | Polyethylene |
| Paragon T8 17125 | Avgol | 16 | pass | 16 | pass | 15 | pass | Polyethylene |

Method for Preparing the Cling Laminates:

The cling films listed in Table 1 were laminated to a nonwoven carrier layer using a Nordson coater/laminator. A pressure sensitive hot melt adhesive was used at an add-on level of 10 grams per square meter and was applied using standard meltblown application equipment. The adhesive was applied to the nonwoven substrate and nipped to the non-cling side of the cling film after an open time of 250 milliseconds. After bonding the two substrates together, the resultant cling laminate was wound onto itself. The adhesive used to prepare the laminates was H2545 and is available from Bostik Findley, Inc. The nonwoven is a standard spunbond polypropylene nonwoven with a basis weight of 14 gsm available from Avgol Nonwoven Industries.

Peel test Method:

The peel test was performed using an Instron tensile tester with a crosshead speed of 92 cm/min (36 inches/minute). A two inch wide sample of laminate was placed with the cling side to the cling side of a second laminate of the same width. A 500 gram roller was used to compress the laminates before testing. The test method used was a standard 180 degree peel test. The average peel strength in grams is reported in the tables. Duplicates were also tested after they were stored in an incubator oven for a period of 24 hours at 38°C (100°F) and 49°C (120°F). The laminates were not bonded during the elevated temperature storage. After aging the samples were tested as before.

Shear Test Method:

A two inch wide sample of cling laminate was placed in contact with a second laminate with the cling sides touching. The overlap area was 5cm by 4cm (two inches by 1-1/2 inches). A standard 500 gram roller was used to compress the structure. A 500 gram weight was used to stress the bonded area in a modified 180 shear configuration while in an incubator oven at 38°C (100°F), i.e. the shear sample was placed around a 15cm (6 inch) core member with the bonded area at about the 9 o'clock position. If the bond held for a period of four hours, it was considered to have passed the test.

Pactiv APM 3-2015 stretch film is available from Pactiv Corporation, 1900 West Field Court, Lake Forest, IL 60045.

Presto films are available from Presto Products Company, P. O. Box 2399, Appleton, WI 54912.

Paragon films are available from Paragon Films, Inc., 3500 West Tacoma, Broken Arrow, OK 74012.

Depending upon the end use requirements of the fastening system on the finished article, the desired peel strength could be higher than that described in Example 1, i.e. up to 393 g/cm (1000g/inch).

### EXAMPLE 2

This example was performed to determine the effect of aging on the peel strength of cling laminates constructed in accordance with the present invention, and to compare the data obtained with that of prior art laminates disclosed in U. S. Patent 5,085,655. Accordingly, the peel test method described in Example 1 was once again performed on 5cm (two inch) wide samples of laminate except using a crosshead speed of 25cm/min (10 inches/minute). The average peel strength in grams is reported in Table 2A initially, after one hour, after one day and after 13 days for laminates made in accordance with the present invention. All samples were stored at room temperature (RT) for the designated time period. These data are then compared to the results reported in Mann et al U. S. Patent 5,085, 655 which describes a prior art laminate using astyrene-ethylene-butylene-styrene (SEBS) block copolymer or an ethylene-propylene rubber (EPR) as an autoadhesive layer. The Mann et al data is reported in Table 2B.

**Table 2A**

| | 25.4cm/min (10"/min) @RT | | | |
|---|---|---|---|---|
| Film | Initial (gm) | 1 Hour (gm) | 1 Day (gm) | 13 Days (gm) |
| Pactiv 2015 | 50 | 19 | 13 | 17 |
| Presto CNC 101515 | 30 | 20 | 22 | 13 |
| Presto CNC 10152 | 13 | 18 | 20 | 20 |
| V1 09015A | 32 | 16 | 14 | 14 |
| T1 28370 Global | 17 | 18 | 17 | 20 |
| T8 17125 | 16 | 21 | 19 | 20 |

**Table 2B**

| (From U.S. Patent 5,085,655) | | | | |
|---|---|---|---|---|
| | 25.4cm/min (10"/min) @RT | | | |
| Film | Initial (gm) | 1 Hour (gm) | 1 Day (gm) | 13 Days (gm) |
| SEBS/SEBS | | | | |
| (Kraton 1657) | N/A | 1364-2043 | 1364-2043 | 2272-2725 |
| EPR/EPR | | | | |
| (Vistalon 719) | N/A | 1590-2271 | 1818-2735 | 2725-3179 |

One can conclude from the above data that the peel strengths of the cling laminates of the present invention tested do not increase to any significant degree during aging, even after 13 days of aging. In contrast, the peel strengths of the prior art laminates increased dramatically over time.

### EXAMPLE 3

As a further comparison, the peel and shear properties of several currently available commercial diaper fastening systems were obtained and tested in the same manner as for Example1. The data is illustrated in Table 3 below. It is to be noted that the peel strength for the tape fastening systems tested are significantly higher than the cling laminates of the present invention and increase substantially during aging for 8 hours at 38°C (100°F).

Peel Values for Various Commercial Fastening Systems:

Several samples of commercially sold diapers were obtained for testing. Two of them used a conventional pressure sensitive tape tab and two used a mechanical fastener system. The same basic peel test that was described previously was used with the following modifications.

The landing zone for each diaper was cut out of the diaper. In the case of the pressure sensitive tape tab, the landing zone consisted of a piece of polypropylene film bonded to the backsheet with a release coating on an outward side. The pressure sensitive tape tab was placed on the release side of the structure and rolled down with a 500 gram roller. In the case of the mechanical fastener, the landing zone consisted of a "loop" material bonded to the backsheet of the diaper. The "hook" tab portion was placed in contact with the "loop" side of the landing zone and rolled down with a 500 gram roller. A 180 degree peel test was performed using the same conditions as noted before. The average peel strength was noted. The pressure sensitive tape tabs were also aged while bonded for eight hours at 38°C (100°F) to see if the peel strength changed. The mechanical fasteners were not aged since the bonds should be unaffected by aging.

**Table 3**

| | Brand | Peel Strength | Type |
|---|---|---|---|
| Sample One - Initial | Toys "R" Us | 181 grams | Tape |
| Sample One - Aged | Toys "R" Us | 393 grams | Tape |
| | | | |
| Sample Two - Initial | Amostra | 260 grams | Tape |
| Sample Two - Aged | Amostra | 827 grams | Tape |
| | | | |
| Sample Three | Huggies Supreme | 42 grams | Mechanical Fastener |
| | | | |
| Sample Four | Pampers Swaddlers | 89 grams | Mechanical Fastener |

Shear tests were also run in accordance with the method set forth in Example 1 on Samples 1 and 2 of Example 3. All samples passed the four hour test initially. No shear testing was performed on the aged samples. However, it is expected that the aged samples would pass.

Although shear testing was not performed on Samples 3 and 4 of Example 3, it is expected that they would pass this test.

### EXAMPLE 4

This example was performed to determine the peel strength of various three layer cling film laminates constructed in accordance with the present invention using a Davis Standard extruder. The peel test method described in Example 1 was used to obtain the data reported in Table 4. In Table 4, the letter "c" refers to a calculated density, or a calculated melt index for the polymer blends.

**Table 4**

| Polymer 1 | Polymer 2 | Run No. | Density | Melt Index | Crystallinity | Comonomer | Peel strength |
|---|---|---|---|---|---|---|---|
| (% polymer) | (% polymer) | | (g/cc) | grams/10 min) | (percent) | (percent) | (grams/inch) |
| | | | | ASTM 1238 | | | |
| | | | | 190C/2.16 kg | | | |
| Affinity EG8200 | | 5 | 0.870 | 5.0 | 19 | 38 | 802 |
| (100%) | | | | | | | |
| | | | | | | | |
| Affinity VP8770 | | 9 | 0.885 | 1.0 | 25 | 30 | 127 |
| (100%) | | | | | | | |
| | | | | | | | |
| Affinity EG8200 | Engage 8842 | 6 | 0.868c | 3.1c | | | 817 |
| (85%) | (15%) | | | | | | |
| Affinity EG8200 | Affinity | 7 | 0.875c | 2.3c | | | 685 |
| (70%) | VP8770 | | | | | | |
| | (30%) | | | | | | |
| | | | | | | | |
| Affinity EG8200 | Affinity | 8 | 0.881c | 1.3c | | | 316 |
| (30%) | VP8770 | | | | | | |
| | (70%) | | | | | | |
| | | | | | | | |
| Affinity PL1880 | Affinity | 13 | 0.892c | 1.3c | | | 33 |
| (30%) | EG8200 | | | | | | |
| | (70%) | | | | | | |
| | | | | | | | |
| Affinity PL1880 | | | 0.902 | 1.0 | 33 | 20 | Not run |
| Engage EG8842 | | | 0.857 | 1.0 | 13 | 45 | Not run |
| | | | | | | | |
| Run 5 to Run 6 | | | | | | | 656 |
| Run 5 to Run 9 | | | | | | | 280 |
| Run 5 to Run 13 | | | | | | | 218 |
| | | | | | | | |
| Run 6 to Run 13 | | | | | | | 340 |
| Run 7 to Run 13 | | | | | | | 259 |
| Run 8 to Run 13 | | | | | | | 154 |
| Run 9 to Run 13 | | | | | | | 75 |

The laminates were produced on a Davis Standard coextrusion unit having three separate layers. The die itself was 10 inch wide and 20 mils thick, fed by three extruders with the screw Length/Diameter ratios of about 15 to 30 to 1. The screw diameters were one inch or one and half inch, with rotational speeds of about 4 to 20 rpm. The melt was heated progressively along three zones with a final temperature of about 190°C to 230°C. The film was extruded onto a chilled metal roll at about 16°C (60°F), then contacted a second roll heated at 38°C (100°F). The line speed was about 12 to 15 meters/min. The film samples were tested a few days afterwards for peel performance.

Top layer was the "cling" film copolymer (or blend of copolymers) as noted above.

Middle layer was the structural layer of the carrier layer for the laminate and comprised Tuflin 7021 which is a LLDPE having a density of 0.914 g/cm³ and a melt index (MI) of 3.2 g/10 min.

Bottom layer was the base layer of the carrier layer for the laminate and comprised a high density polyethylene with a density of 0.934 g/cm³ and a MI of 2.7 g/10 min.

Each film layer was 1 mil thick

Affinity and Tuflin grades available from Dow Chemical Co.

Engage grades available from DuPont Dow Elastomers

The high density polyethylene used for the bottom layer is available from Atofina Petrochemicals

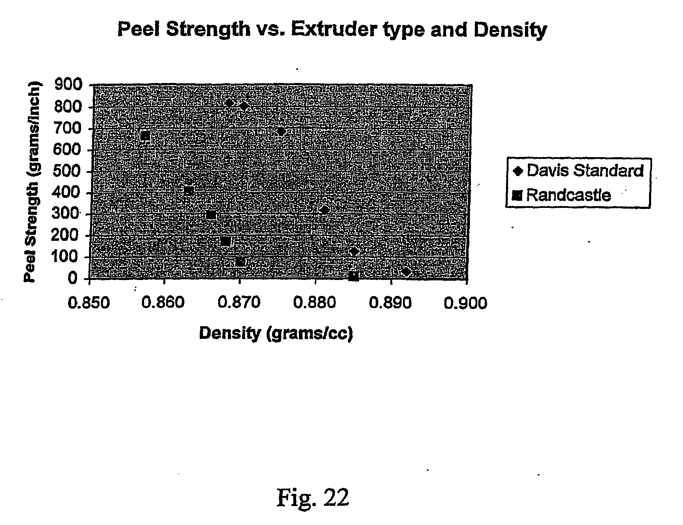

The data illustrate that the peel strengths for the present cling film laminates increase as the density decreases, and the cling film laminates are more than adequate for use in fastening and bonding systems as described herein whereas prior art cling films did not have sufficient peel strength. The relationship between density and peel strength is also illustrated in Fig. 22.

### EXAMPLE 5

This example was performed to determine the peel strength of various cling layers constructed in accordance with the present invention using a Randcastle Monolayer extruder. The peel test method described in Example 1 was used to obtain the data reported in Table 5. In Table 5, the letter "c" refers to a calculated density, or a calculated melt index for the polymer blends.

**Table 5**

| Polymer 1 | 1 polymer 2 | Density | Melt Index | Crystallinity | Comonomer | Peel Strength |
|---|---|---|---|---|---|---|
| (% polymer) | (% polymer) | (g/cc) | | | (percent) | (grams/inch) |
| | | | (grams/ 10 min) | (percent) | | |
| Affinity EG8200 (100%) | | 0.87 | 5 | 19 | 38 | 75 |
| Engage 8842 (100%) | | 0.857 | 1 | 13 | 45 | 665 |
| Affinity VP8770 (100%) | | 0.885 | 1 | 25 | 30 | 11 |
| Affinity EG8200 (85%) | Affinity 8842(15%) | 0.868c | 3.1c | | | 171 |
| Affinity BG8200 (70%) | Affinity 8842 (30%) | 0.866c | 2.3c | | | 294 |
| Affinity EG8200 (50%) | Affinity 8842 (50%) | 0.863c | 1.7c | | | 405 |

Films were produced on a Randcastle lab-scale monolayer extrusion unit, and each film was 3 mils thick. The die itself was 6 inch wide fed by one extruder with a screw Length/Diameter ratio of about 20 to 1. The screw speed was about 4 to 25 rpm. The melt was heated along four zones with a final temperature of about 180°C to 240°C. The film first met a chilled metal roll at about 16°C (60°F). The line speed was about 0.4 to 1.5 meters/min. Film samples were then tested a few days afterwards for peel performance.

The data illustrate that the peel strengths for the present cling film laminates increase as density decreases, and the cling film laminates are more than adequate for use in the fastening and bonding systems as described herein. This relationship between density and peel strength is further illustrated in Fig. 22.

It should be noted that in the above Example 5, Affinity VP 8770 is an example of the substantially non-stretchable, self-supporting sheet or monolayer embodiment of the cling film fastener of the present invention. In addition, it should be noted that the cling surfaces of the films made on the Randcastle extruder unit (Example 5) were not as smooth as the cling surfaces of the films made on the Davis Standard unit (Example 4) resulting in the same copolymer (e. g. Affinity VP 8770) having a higher peel strength when its cling surface is smooth and untextured. This demonstrates that varying the texture of the cling surface results in different peel strengths for the same density copolymer.

### EXAMPLE 6

This example was performed to determine the peel strength of various three layer cling film laminates constructed in accordance with the present invention using propylene-based copolymers ("Versify" from Dow Chemical Company), and to compare these peel strengths with similar laminates constructed of ethylene based copolymers("Affinity" from Dow Chemical Company). The peel test method described in Example 1 was used to obtain the data reported in Tables 6A and 6B. The following copolymers were used for the cling layer of the laminates:

| Commercial Reference | Supplier | Additives Anti-Block Anti-Slip | MFI (g/10 min) ASTM 1238 | Density (g/cc) | Type of Polymer/Technology | Features |
|---|---|---|---|---|---|---|
| Affinity | Dow Chemical | None except Aox | 0.5 | 0.868 | Ethylene/Octene metallocene | Narrow |
| EG8150 | | | | | | MWD |
| | | | | | | PI=2to2.1 |
| Affinity | Dow Chemical | None except Aox | 3 | 0.870 | Ethylene/Octene metallocene | Narrow |
| EG8200 | | | | | | MWD |
| | | | | | | PI=2to2.1 |
| Versify | Dow Chemical | | 2 | 0.859 | Propylene/Ethylene metallocene | Contains 15% of C2 |
| DE 2400.00 | | | | | | |
| Versify | Dow Chemical | | 2 | 0.876 | Propylene/Ethylene metallocene | Contains 9% of C2 |
| DE 2200.00 | | | | | | |

The 3-layer film laminates were produced on a Davis Standard coextrusion unit having three separate feed circuits. The die itself was 10 inch wide and 20 mils thick, fed by three extruders with the screwLength/Diameter ratios of about 15 to 30 to 1. The screw diameters were one inch or one and half inch, with rotational speeds of about 4 to 20 rpm. The melt was heated progressively along three zones with a final temperature of about 190°C to 230°C. The film structure was extruded onto a chilled metal roll at about 16°C (60°F), then contacted a second roll heated at 38°C (100°F). The line speed was about 12 to 15 meters/min.

The film structure consisted in a 25µm (1-mil) thick layer of the cling material, then a 1-mil-thick layer of a medium density PE (polyethylene), then a 13µm (0.5-mil) thick layer of high density PE (polyethylene). Rolls were made laminating the cling surfaces with some PET (polyethylene terephthalate) film as a protective sheet against contamination and potential blocking.

The peel data is reported in Table 6A and demonstrate that although the peel force of the propylene-based cling layer is less than that of the ethylene-based cling layer, the propylene-based cling layer still provides sufficient peel strength, depending on the desired end use application for the laminate.

**Table 6A**

| | Affinity | Affinity | Versify | Versify |
|---|---|---|---|---|
| | EG8200 | EG8150 | 2400 | 2200 |
| 1st immediate peel in g/cm (g/in) | 202 (512) | 104 (263) | 76 (194) | 6 (15) |
| Std dev g/cm (g/in) on 5 samples | 15 (39) | 15 (38) | 17 (42) | 2 (5) |

Table 6B provides a comparison of peel strengths for the cling layers after one peel, three peels, and five successive peels to demonstrate the resealability of the ethylene-based and propylene-based cling layers. The data illustrate that the propylene-based cling layers maintain their peel strengths (and this their ability to reseal itself) even after five successive peels whereas the ethylene-based cling layers tend to lose their peel strengths at a much greater rate as the number of successive peels increase.

**Table 6B**

| | Affinity | Affinity | Versify | Versify |
|---|---|---|---|---|
| Immediate Successive Peels | EG8200 | EG8150 | 2400 | 2200 |
| 1st immediate peel in g/cm (g/in) | 202 (512) | 104 (263) | 76 (194) | 6 (15) |
| Std dev g/cm (g/in) on 5 samples | 15 (39) | 15 (38) | 17 (42) | 2 (5) |
| 3rd immediate peel in g/cm (g/in) | 44 (113) | 76 (194) | 84 (213) | 6 (14) |
| Std dev g/cm (g/in) on 5 samples : | 9 (23) | 4 (9) | 4 (9) | 2 (4) |
| 5th immediate peel in g/cm (g/in) | 18 (45) | 20 (52) | 75 (190) | |
| Std dev g/cm (g/in) on 5 samples | 6 (15) | 8 (21) | 5(13) | |

## Claims

1. A cling film fastener system having a fastening component comprising:
a cling layer that does not stretch more than 50% having an inner surface and an outer cling surface, said cling layer comprising a polyolefin copolymer, said polyolefin copolymer selected from the group consisting of a metallocene or single site catalyzed ethylene-based copolymer of ethylene and a C₃ to C₁₈ alpha-olefin comonomer having at least 20% by weight of said comonomer, a metallocene or single site catalyzed propylene-based copolymer of propylene and a C₂ to C₁₈ alpha-olefin comonomer having at least 5% by weight of said comonomer, and a blend of the ethylene-based copolymers, the propylene-based copolymers, or one or more of said ethylene-based copolymers with one or more of the propylene-based copolymers.

2. The fastener system of claim 1 wherein said ethylene-based copolymer has 20% to 50% by weight of said comonomer, preferably has 25% to 50% by weight of said comonomer, and most preferably has 30% to 50% by weight of said comonomer.

3. The fastener system of claim 1 wherein said ethylene-based copolymer has a density of 0.89g/cm³ or less, preferably has a density of 0.89g/cm³ to 0.85g/cm³, more preferably has a density of 0.88g/cm³ to 0.85g/cm³, and most preferably has a density of 0.875g/cm³ to 0.85g/cm³.

4. The fastener system of claim 1 wherein said ethylene-based copolymer has a melt index of 100g/10min. or less at 190°C, preferably a melt index of 50g/10min. or less at 190°C, and said propylene-based copolymer has a melt index of 100g/10min. or less at 230°C, preferably a melt index of 50g/10min. or less at 230°C.

5. The fastener system of claim 1 wherein the alpha-olefin comonomer of said ethylene-based copolymer comprises octene.

6. The fastener system of claim 1 wherein said propylene-based copolymer has 5% to 30% by weight of said comonomer, and preferably has 5% to 15% by weight of said comonomer.

7. The fastener system of claim 1 wherein the alpha-olefin comonomer of said propylene-based copolymer comprises ethylene.

8. The fastener system of claim 1 wherein said blend has a calculated density of 0.89g/cm³ or less, preferably said blend has a calculated density of 0.89g/cm³ to 0.85g/cm³, more preferably said blend has a calculated density of 0.88g/cm³ to 0.85g/cm³, and most preferably said blend has a calculated density of 0.875g/cm³ to 0.85g/cm³.

9. The fastener system of claim 1 wherein said fastening component further comprising a carrier layer having first and second opposite sides, said first side bonded to the inner surface of said cling layer forming a cling film laminate structure.

10. The fastener system of claim 9 wherein said carrier layer comprises a nonwoven, or said carrier layer comprises a thermoplastic film selected from the group consisting of a polyolefin, a copolymer of ethylene and C₃ to C₁₈ olefins, a polyester, a polyamide, a polysulfone, an acrylic polymer, a polystyrene, a polyurethane, a polycarbonate, a halogenated polymer, a cellulosic, a polyacrylonitrile, an ethylene vinyl acetate, and an ionomer based on sodium or zinc salts of ethylene/methacrylic acid.

11. The fastener system of claim 10 wherein said polyolefin is a polyethylene, polypropylene or polybutylene.

12. The fastener system of claim 10 wherein said polyamide is nylon.

13. The fastener system of claim 10 wherein said acrylic polymer is polyethylene methyl acrylic acid, polyethylene-n-butyl acrylate, polyethylene ethyl acrylate, or polyethylene methyl acrylate.

14. The fastener system of claim 10 wherein said halogenated polymer is polyvinylchloride or polyvinylidene chloride.

15. The fastener system of claim 1 or claim 9 wherein said cling layer or said cling film laminate structure stretches 25% or less from its original non-stretched configuration, preferably 10% or less from its original non-stretched configuration.

16. The fastener system of claim 9 wherein said carrier layer is substantially non-stretchable from its original configuration, and preferably stretches 25% or less from its original configuration and most preferably stretches 10% or less from its original configuration.

17. The fastener system of claim 1 wherein said cling layer comprises a self-supporting monolayer of said polyolefin copolymer.

18. The fastener system of claim 1 wherein the inner surface of said cling layer also exhibits cling.

19. The fastener system of claim 9 further including a target surface and a bond between the second side of said carrier layer and said target surface, said bond comprises a hot melt adhesive coated on said second side or said target surface, or said bond comprises a pressure sensitive adhesive coated on said second side or said target surface, or said bond comprises a hook and loop reclosable fastener, or said bond comprises a mushroom-shaped reclosable fastener, or said bond comprises an ultrasonic weld, or said bond comprises a mechanical bond, or said bond comprises a thermal bond.

20. The fastener system of claims 1, 9 or 17 incorporated into a package, envelope, tape, or disposable soft goods article.

21. The fastener system of claim 20 wherein the disposable soft goods article is a disposable diaper having first and second waist portions, and said fastener system comprises an ear, tab or tape that joins said first and second waist portions when the diaper is configured in said usable position.

22. The fastener system of claim 21 wherein said fastener system is bonded to at least one of said waist portions, or is integrally formed as part of at least one of said waist portions.

23. The fastener system of claim 20 wherein the disposable soft goods article is a feminine napkin having a pair of wings extending laterally outwardly in opposite directions from the absorbent substrate, and said fastener system joins said wings when the feminine napkin is configured in said usable position.

24. The fastener system of claim 23 wherein said fastener system is bonded to at least one of said wings, or is integrally formed as part of at least one of said wings.

25. The fastener system of claim 20 wherein the disposable soft goods article is a surgical drape, or a hospital gown, or a hospital pad.

26. The fastener system of claim 20 wherein the package comprises a box, or a flexible wrapper, or a blister package.

## Patentansprüche

1. Haftfilmbefestigungssystem mit einer Befestigungskomponente, **gekennzeichnet durch**:
eine Haftschicht, die sich nicht mehr als 50 % dehnt und mit einer Innenfläche und einer Haftaußenfläche, wobei die Haftschicht ein Polyolefin Copolymer aufweist, das aus einer Gruppe ausgewählt worden ist, die sich aus einem metallocenen oder "single-site" katalysierten Ethylen-basierten Copolymer aus Ethylen und einem C₃ bis C₁₈ Alpha-Olefin Comonomer mit mindestens 20 % Massenanteil an dem Comonomer, einem metallocenen oder "single-site" katalysierten Propylen-basierten Copolymer aus Propylen und einem C₂ bis C₁₈ Alpha-Olefin Comonomer mit mindestens 5 % Massenanteil an dem Comonomer, und einer Mischung aus dem Ethylen-basierten Copolymer, dem Propylen-basierten Copolymer, oder einem oder mehreren der Ethylen-basierten Coploymere mit einem oder mehreren der Propylen-basierten Coploymere zusammensetzt.

2. Befestigungssystem nach Anspruch 1, **dadurch gekennzeichnet, dass** das Ethylen-basierte Copolymer einen Massenanteil von 20 % bis 50 %, vorzugsweise einen Massenanteil von 25 % bis 50 %, und am meisten bevorzugt einen Massenanteil von 30 % bis 50 %, an dem Comonomer aufweist.

3. Befestigungssystem nach Anspruch 1, **dadurch gekennzeichnet, dass** das Ethylen-basierte Copolymer eine Dichte von 0,89 g/cm³ oder weniger, vorzugsweise eine Dichte von 0,89 g/cm³ bis 0,85 g/cm³, besonders bevorzugt eine Dichte von 0,88 g/cm³ bis 0,85 g/cm³, und am meisten bevorzugt eine Dichte von 0,875 g/cm³ bis 0,85 g/cm³, aufweist.

4. Befestigungssystem nach Anspruch 1, **dadurch gekennzeichnet, dass** das Ethylen-basierte Copolymer einen Schmelzindex von 100g/10min oder weniger bei 190°C, vorzugsweise einen Schmelzindex von 50g/10min oder weniger bei 190°C, aufweist, und dass das Propylen-basierte Copolymer einen Schmelzindex von 100g/10min oder weniger bei 230°C, vorzugsweise einen Schmelzindex von 50g/10min oder weniger bei 230°C, aufweist.

5. Befestigungssystem nach Anspruch 1, **dadurch gekennzeichnet, dass** das Alpha-Olefin Comonomer des Ethylen-basierten Copolymers Octen aufweist.

6. Befestigungssystem nach Anspruch 1, **dadurch gekennzeichnet, dass** das Propylen-basierte Copolymer einen Massenanteil von 5 % bis 30 %, vorzugsweise einen Massenanteil von 5 % bis 15 %, an dem Comonomer aufweist.

7. Befestigungssystem nach Anspruch 1, **dadurch gekennzeichnet, dass** das Alpha-Olefin Comonomer des Propylen-basierten Copolymers Ethylen aufweist.

8. Befestigungssystem nach Anspruch 1, **dadurch gekennzeichnet, dass** die Mischung eine berechnete Dichte von 0,89 g/cm³ oder weniger, vorzugsweise eine berechnete Dichte von 0,89 g/cm³ bis 0,85 g/cm³, besonders bevorzugt eine berechnete Dichte von 0,88 g/cm³ bis 0,85 g/cm³, und am meisten bevorzugt eine berechnete Dichte von 0,875 g/cm³ bis 0,85 g/cm³, aufweist.

9. Befestigungssystem nach Anspruch 1, **dadurch gekennzeichnet, dass** die Befestigungskomponente des Weiteren eine Trägerschicht mit einer ersten und einer zweiten gegenüberliegenden Seite aufweist, wobei die erste Seite mit der Innenfläche der Haftschicht zum Ausbilden einer Haftfilmverbundstruktur verbunden ist.

10. Befestigungssystem nach Anspruch 9, **dadurch gekennzeichnet, dass** die Trägerschicht einen Vliesstoff oder eine thermoplastische Schicht aufweist, die aus einer Gruppe ausgewählt worden ist, die sich aus Polyolefin, einem Copoylmer aus Ethylen und C₃ bis C₁₈ Olefinen, Polyester, Polyamid, Polysulfon, Acrylpolymer, Polystyrol, Polyurethan, Polycarbonat, halogeniertes Polymer, Zellulose, Polyacrylnitril, Ethylenvinylacetat, und aus einem Ionomer basierend auf Natrium- oder Zinksalzen der Ethylen/Methacrylsäure zusammensetzt.

11. Befestigungssystem nach Anspruch 10, **dadurch gekennzeichnet, dass** das Polylefin Polyethylen, Polypropylen oder Polybutylen ist.

12. Befestigungssystem nach Anspruch 10, **dadurch gekennzeichnet, dass** das Polyamid Nylon ist.

13. Befestigungssystem nach Anspruch 10, **dadurch gekennzeichnet, dass** das Acrylpolymer Polyethylen-Methylacrylsäure, Polyethylen-n-Butylacrylat, Polyethylen-Ethylacrylat oder Polyethylen-Methylacrylat ist.

14. Befestigungssystem nach Anspruch 10, **dadurch gekennzeichnet, dass** das halogenierte Polymer Polyvinylchlorid oder Polyvinylidenchlorid ist.

15. Befestigungssystem nach Anspruch 1 oder 9, **dadurch gekennzeichnet, dass** sich die Haftschicht oder die Haftfilmverbundstruktur um 25 % oder weniger, insbesondere um 10 % oder weniger, aus ihrer ursprünglichen nicht gedehnten Anordnung dehnt.

16. Befestigungssystem nach Anspruch 9, **dadurch gekennzeichnet, dass** die Trägerschicht aus ihrer ursprünglichen Anordnung im Wesentlichen nicht dehnbar ist, und sich vorzugsweise um 25 % oder weniger aus ihrer ursprünglichen Anordnung dehnt und am meisten bevorzugt sich um 10 % oder weniger aus ihrer ursprünglichen Anordnung dehnt.

17. Befestigungssystem nach Anspruch 1, **dadurch gekennzeichnet, dass** die Haftschicht eine selbst tragende Monoschicht aus Polyolefin Copolymer aufweist.

18. Befestigungssystem nach Anspruch 1, **dadurch gekennzeichnet, dass** die Innenfläche der Haftschicht ebenfalls Hafteigenschaften aufweist.

19. Befestigungssystem nach Anspruch 9, **gekennzeichnet durch** eine Zielfläche und einer Verbindung zwischen der zweiten Seite der Trägerschicht und der Zielfläche, wobei die Verbindung einen auf der zweiten Seite oder der Zielfläche aufgebrachten Schmelzklebstoff, einen auf der zweiten Seite oder der Zielfläche aufgebrachten drucksensitiven Klebstoff, einen wieder verschließbaren Klettverschluss, einen wieder verschließbaren Druckknopfverschluss, eine Ultraschallschweißverbindung, oder eine mechanische oder eine thermische Verbindung aufweist.

20. Befestigungssystem nach Anspruch 1, 9 oder 17, **dadurch gekennzeichnet, dass** dieses in eine Verpackung, einen Umschlag, einen Klebestreifen oder in einen Einwegartikel eingearbeitet ist.

21. Befestigungssystem nach Anspruch 20, **dadurch gekennzeichnet, dass** der Einwegartikel eine Einwegwindel mit einem ersten Taillenbereich und einem zweiten Taillenbereich ist, wobei das Befestigungssystem eine Lasche, eine Schlaufe oder einen Klebestreifen aufweist, der bzw. die den ersten und zweiten Taillenbereich verbindet, wenn die Windel in dem Verwendungszustand konfiguriert ist.

22. Befestigungssystem nach Anspruch 21, **dadurch gekennzeichnet, dass** das Befestigungssystem mindestens an einem Taillenbereich befestigt ist, oder einstückig als ein Teil mindestens eines Taillenbereiches ausgebildet ist.

23. Befestigungssystem nach Anspruch 20, **dadurch gekennzeichnet, dass** der Einwegartikel eine Damenbinde mit einem Paar Flügeln ist, die sich seitlich auswärts in entgegen gesetzte Richtungen von dem absorbierenden Substrat erstrecken, und dass das Befestigungssystem die Flügel verbindet, wenn die Damenbinde in dem Verwendungszustand konfiguriert ist.

24. Befestigungssystem nach Anspruch 23, **dadurch gekennzeichnet, dass** das Befestigungssystem mindestens an einem der Flügel befestigt ist, oder einstückig als ein Teil mindestens eines der Flügel ausgebildet ist.

25. Befestigungssystem nach Anspruch 20, **dadurch gekennzeichnet, dass** der Einwegartikel ein chirurgisches OP-Abdecktuch oder ein Klinkkittel oder ein Kliniktupfer ist.

26. Befestigungssystem nach Anspruch 20, **dadurch gekennzeichnet, dass** die Verpackung eine Box oder eine flexible Hülle oder eine Blisterverpackung aufweist.

## Revendications

1. Système de fixation à pellicule adhésive ayant un composant de fixation comprenant :
une couche adhésive qui ne s'étire pas plus de 50% ayant une surface interne et une surface externe adhésive, cette couche adhésive comprenant un copolymère de polyoléfine, ledit copolymère de polyoléfine étant choisi dans le groupe constitué par un copolymère d'éthylène à base d'éthylène catalysé par un métallocène ou simple site, un copolymère de propylène à base de propylène catalysé par un métallocène ou simple site et un comonomère d'alpha oléfine en C₂ à C₁₈ ayant au moins 5% en poids dudit comonomère et un mélange des copolymères à base d'éthylène, des copolymères à base de propylène ou un ou plusieurs desdits copolymères à base d'éthylène avec un ou plusieurs desdits copolymères à base de propylène.

2. Système de fixation selon la revendication 1, dans lequel ledit copolymère à base d'éthylène a entre 20% et 50% en poids dudit comonomère, de préférence, entre 25% et 50% en poids dudit comonomère et de façon plus préférée entre 30% et 50% en poids dudit comonomère.

3. Système de fixation selon la revendication 1, dans lequel ledit copolymère à base d'éthylène a une densité de 0,89g/cm³ ou moins, de préférence a une densité de 0,89g/cm³ à 0,85g/cm³, de façon plus préférée a une densité de 0,88g/cm³ à 0,85g/cm³ et de façon la plus préférée a une densité de 0,875g/cm³ à 0,85g/cm³.

4. Système de fixation selon la revendication 1, dans lequel ledit copolymère à base d'éthylène a un indice de fusion de 100g/10mn ou moins à 190°C, de préférence a un indice de fusion de 50g/10mn ou moins à 190°C et ledit copolymère à base de propylène a un indice de fusion de 100g/10mn ou moins à 230°C, de préférence un indice de fusion de 50g/10mn ou moins à 230°C.

5. Système de fixation selon la revendication 1, dans lequel le comonomère dudit copolymère à base d'éthylène comprend de l'octène.

6. Système de fixation selon la revendication 1, dans lequel ledit copolymère à base de propylène a entre 5% et 30% en poids dudit comonomère et de préférence a entre 5% et 15% en poids dudit comonomère.

7. Système de fixation selon la revendication 1, dans lequel le comonomère d'alpha-oléfine dudit copolymère à base de propylène comprend de l'éthylène.

8. Système de fixation selon la revendication 1, dans lequel ledit mélange a une densité calculée de 0,89g/cm³ ou moins, de préférence ledit mélange a une densité calculée de 0,89g/cm³ à 0,85/cm³, de façon plus préférée ledit mélange a une densité calculée de 0,88g/cm³ à 0,85g/cm³ et de façon la plus préférée ledit mélange a une densité calculée de 0,875g/cm³ à 0,85g/cm³.

9. Système de fixation selon la revendication 1, dans lequel ledit composant de fixation comprend une couche de support ayant un premier et un second côtés opposés, ledit premier côté relié à la surface interne de ladite couche adhésive formant une structure laminée d'une pellicule adhésive.

10. Système de fixation selon la revendication 9, dans lequel ladite couche de support comprend un non-tissé ou ladite couche de support comprend une pellicule thermoplastique choisie dans le groupe constitué par une polyoléfine, un copolymère d'éthylène et des oléfines de C₃ à C₁₈, un polyester, une polyamide, une polysulfone, un polymère acrylique, un polystyrène, un polyuréthane, un polycarbonate, un polymère halogéné, une cellulosique, un polyacrylonitrile, un acétate d'éthylène vinyle et un oinomère à base de sels de sodium et de zinc d'acide éthylène/méthacrylique.

11. Système de fixation selon la revendication 10, dans lequel ladite polyoléfine est du polyéthylène, du polypropylène ou du polybutylène.

12. Système de fixation selon la revendication 10 dans lequel ledit polyamide est du nylon.

13. Système de fixation selon la revendication 10, dans lequel ledit polymère acrylique est de l'acide polyéthylène méthyle acrylique, de l'acrylate de polyéthylène-n butyle, de l'acrylate de polyéthylène éthyle, ou de l'acrylate de polyéthylène méthyle.

14. Système de fixation selon la revendication 10 dans lequel ledit polymère halogéné est du chlorure de polyvinyle ou du chlorure de polyvinylidène.

15. Système de fixation selon la revendication 1 ou la revendication 9, dans lequel ladite couche adhésive ou ladite structure laminée de couche adhésive s'étire de 20% ou moins par rapport à sa configuration d'origine non-étirée, de préférence 10% ou moins par rapport à sa configuration initiale non-étirée.

16. Système de fixation selon la revendication 9, dans lequel ladite couche de support est sensiblement non étirable à partir de sa configuration d'origine et de préférence s'étire de 25% ou moins par rapport à sa configuration d'origine et de façon plus préférée s'étire de 10% ou moins par rapport à sa configuration d'origine.

17. Système de fixation selon la revendication 1, dans lequel ladite couche adhésive comporte une monocouche auto-portante dudit copolymère de polyoléfine.

18. Système de fixation selon la revendication 1, dans lequel la surface interne de ladite couche adhésive est également adhésive.

19. Système de fixation selon la revendication 9, comprenant une surface cible et une liaison entre le second côté de ladite couche de support et ladite surface cible, ladite liaison comprenant une adhésif fusible à chaud revêtant ledit second côté ou ladite surface cible, ou ladite liaison comportant un adhésif sensible à la pression revêtant ledit second côté ou ladite surface cible, ou ladite liaison comportant une fixation refermable à crochets et boucles, ou ladite liaison comportant une fixation refermable en forme de champignon ou ladite liaison comportant une soudure ultrasonique, ou ladite liaison comportant une liaison mécanique, ou ladite liaison comportant une liaison thermique.

20. Système de fixation selon les revendications 1, 9 ou 17 incorporé dans un emballage, une enveloppe, bande ou article de produit mou jetable.

21. Système de fixation selon la revendication 20 dans lequel l'article de produit mou jetable est une couche jetable ayant une première et une seconde parties ventrales et ledit système de fixation comprend une oreille, un patin ou bande qui relie lesdites première et seconde parties ventrales lorsque la couche est configurée dans la position d'utilisation.

22. Système de fixation selon la revendication 21, dans lequel ledit système de fixation est relié à au moins l'une desdites parties ventrales, ou est formé d'une seule pièce en tant que partie de l'une au moins desdites parties ventrales.

23. Système de fixation selon la revendication 20, dans lequel l'article de produit mou jetable est une serviette féminine ayant une paire d'ailes s'étendant latéralement vers l'extérieur dans des directions opposées à partie de substrat absorbant et ledit système de fixation relie lesdites ailes lorsque ladite serviette féminine est configurée dans la position d'utilisation.

24. Système de fixation selon la revendication 23, dans lequel ledit système de fixation est relié à au moins l'une desdites ailes, ou est formé d'une seule pièce en tant que partie d'au moins une desdites ailes.

25. Système de fixation selon la revendication 20, dans lequel l'article de produit mou jetable est un drap chirurgical, une blouse d'hôpital ou un patin d'hôpital.

26. Système de fixation selon la revendication 20 dans lequel l'emballage comprend une boîte, une enveloppe flexible ou un emballage sous forme de blister.
